# EUROPEAN PATENT SPECIFICATION

(11) **EP 1 948 684 B1**
(45) Date of publication and mention of the grant of the patent: **27.01.2016**
(21) Application number: 06818633.7
(22) Date of filing: 17.11.2006
(51) Int. Cl.: C07K 14/435

(54) **PRODUCTION OF RECOMBINANT COLLAGEN LIKE PROTEINS**
REKOMBINANTE MUSCHEL BYSSUS PROTEINE
PROTEINE RECOMBINANTE DU BYSSUS DE MOULE

(30) Priority: 17.11.2005 EP 05025131
(43) Date of publication of application: 30.07.2008
(73) Proprietor: AMSilk GmbH, 82152 Planegg/Martinsried (DE)
(72) Inventor: SCHEIBEL, Thomas, 95448 Bayreuth (DE)
(74) Representative: Zwicker, Jörk
(86) International application number: PCT/EP2006/011061
(87) International publication number: WO 2007/057207

(56) References cited:
- COYNE KATHYRN J ET AL: "Extensible collagen in mussel byssus: A natural block copolymer" SCIENCE (WASHINGTON D C), vol. 277, no. 5333, 1997, pages 1830-1832, XP002386502 ISSN: 0036-8075
- QIN XIAO-XIA ET AL: "Tough tendons. Mussel byssus has collagen with silk-like domains" JOURNAL OF BIOLOGICAL CHEMISTRY, vol. 272, no. 51, 19 December 1997 (1997-12-19), pages 32623-32627, XP002386506 ISSN: 0021-9258
- WAITE J HERBERT ET AL: "The peculiar collagens of mussel byssus" MATRIX BIOLOGY, vol. 17, no. 2, June 1998 (1998-06), pages 93-106, XP002386504 ISSN: 0945-053X
- COYNE KATHRYN J ET AL: "In search of molecular dovetails in mussel byssus: From the threads to the stem" JOURNAL OF EXPERIMENTAL BIOLOGY, vol. 203, no. 9, May 2000 (2000-05), pages 1425-1431, XP002386505 ISSN: 0022-0949
- LUCAS JARED M ET AL: "A molecular, morphometric and mechanical comparison of the structural elements of byssus from Mytilus edulis and Mytilus galloprovincialis" JOURNAL OF EXPERIMENTAL BIOLOGY, vol. 205, no. 12, June 2002 (2002-06), pages 1807-1817, XP002386503 ISSN: 0022-0949
- OLSEN D ET AL: "Recombinant collagen and gelatin for drug delivery" ADVANCED DRUG DELIVERY REVIEWS, AMSTERDAM, NL, vol. 55, no. 12, 28 November 2003 (2003-11-28), pages 1547-1567, XP002368792 ISSN: 0169-409X
- BAEZ JULIO ET AL: "Recombinant microbial systems for the production of human collagen and gelatin" APPLIED MICROBIOLOGY AND BIOTECHNOLOGY, vol. 69, no. 3, December 2005 (2005-12), pages 245-252, XP002428474 ISSN: 0175-7598
- DEMING T J ET AL: "MUSSEL BYSSUS AND BIOMOLECULAR MATERIALS", CURRENT OPINION IN CHEMICAL BIOLOGY, CURRENT BIOLOGY LTD, LONDON, GB LNKD- DOI:10.1016/S1367-5931(99)80018-0, vol. 3, 1 January 1999 (1999-01-01), pages 100-105, XP002938829, ISSN: 1367-5931
- GOSLINE J ET AL: "ELASTIC PROTEINS: BIOLOGICAL ROLES AND MECHANICAL PROPERTIES", PHILOSOPHICAL TRANSACTIONS. ROYAL SOCIETY OF LONDON.BIOLOGICAL SCIENCES, ROYAL SOCIETY, LONDON, GB LNKD- DOI:10.1098/RSTB.2001.1022, vol. 357, no. 1418, 28 February 2002 (2002-02-28), pages 121-132, XP009006960, ISSN: 0962-8436

## Description

The present invention is directed to a yeast cell for producing a recombinant mussel byssus protein. The present invention is further directed to a kit of parts or a co-expression system for use in the production of such a protein and to a method of producing said recombinant protein and a thread made therefrom.

Marine mussels are found in the turbulent habitat of the inter-tidal zone and here, marine mussels have been very successful in colonizing rocks, which are exposed to wind and waves. This success is partially due to a unique anchorage by which they fix themselves on the solid surfaces of the rocks. A part of this anchorage is a fibrilar structure, known as "byssus" or also known as "mussel silk". The byssus provides mussels with the necessary tenacity to survive the incessant buffeting of waves by attaching to rocks or hard surfaces.

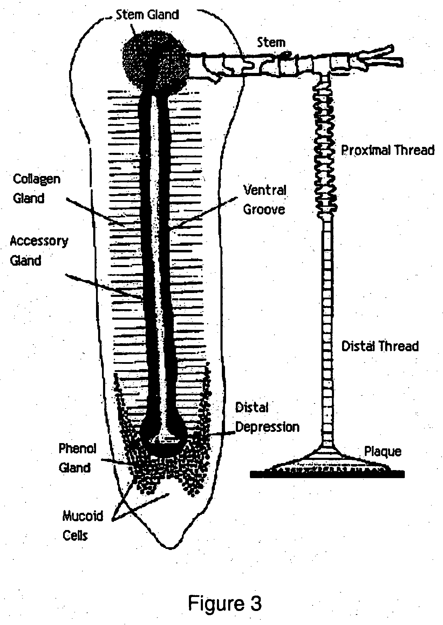

The mussel byssus is completely consisting of extra-cellular matrix which is forming a bundle of short threads that resemble tiny tendons [2]. Byssus threads show unusual mechanical properties, since they resemble soft rubber at one end and rigid nylon at the other and these properties are found with a seamless and gradual transition [4]. Byssal threads are also elastomeric: they are able to withstand significant deformations without rupture and can return to their original state, when the stress is removed [5]. At the distal end, the byssus threads are fixed by adhesive plaques at the rock. At the proximal ends, the byssus threads are combined to a so-called byssus stem, which is anchored at the base of the mussel foot (see Figure 3).

The byssus threads of marine mussels are elastomeric fibers with a great capacity of absorbing and dissipating energy. Up to 70% of the total absorbed energy can be dissipated in the byssus. In *Mytilus* species (*M. edulis* and *M. galoprovincialis*), each new thread has dimensions of a few centimeters in length and less than 0.1 cm in diameter and is produced in ca. 5 minutes in the ventral groove of the foot by a process akin to reaction injection molding [3].

Morphologically, the byssus is divided into four sections (from proximal to distal): root, stem, thread and plaque or pad. Furthermore, the thread is further subdivided into proximal and distal portions according to appearance, i.e. smooth arid stiff for the distal, soft and weaker for the proximal portion.

Byssus threads are elastomeric. The Young's modulus is low (in the range of from 10-500 MPa), the extensibility can be as high as 200% and there is restorative recall. In common with other protein elastomers as elastin, resiline and abductine, byssus threads are quite tough. Thoughness and energy dissipation are both crucial properties for holdfasts. Energy dissipation in fibers subjected to cyclic stress-strain-analysis is frequently normalized with respect to the total absorbed energy and reported as hysteresis or percentage hysteresis.

The stress-strain cycle for one thread has been dissected into separate mechanical contributions for the distal and proximal portions of the thread. As mentioned above, of these, the distal portion is stronger, stiffer and superior at damping whereas the proximal portion is softer and weaker with a lower, but still significant hysteresis.

The mechanical properties of byssus threads are further complicated by time- and strain-dependent behavior. It was demonstrated that, when strained beyond its yield point, the distal portion exhibited a schematic stress softening, i.e. the initial modulus of the second cycle was reduced to about 20% of the modulus in the first cycle (500-80 MPa). The complete recovery of the modulus of the first cycle was slow, e.g. longer than 24h but significant partial recovery can occur within 1h (30% of the original values). The proximal portion also shows a tendency to change stiffness with cyclic loading. In this case, there is strain-stiffening from an initial modulus of 35 MPa to an asymptotic leveling at 50 MPa, an increase of about 40%.

MASCOLO and WAITE (1986) first identified chemical gradients in byssus threads in *Mytilus.* After treatment of the threads with pepsin, two pepsin-resistant collagen fragments, called ColP and ColD, having molecular weights of 50 kDa and 60 kDa, respectively were identified. ColP can be found predominantly in the proximal area and is hardly to be found in the distal area. In contrast, the amount of ColD increases in the distal part to approximately 100% (LUCAS et al., 2002; QIN & WAITE, 1995). In the byssus thread as well as in the mussel foot, there is a further collagen-like protein which takes part in the construction of the thread structure. This additional protein is called ColNG (NG = no gradient), and is, in contrast to ColD and ColP, evenly distributed throughout the whole thread. Its physiological function presumably is being an adapter between the two other thread collagens (QIN & WAITE, 1998).

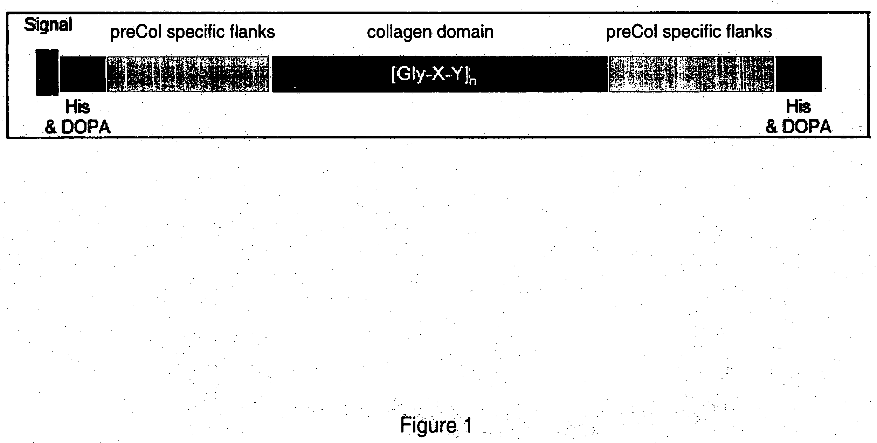

The Pepsin-cleaved fragments ColD and P originate from the so-called preCollagens P and D. Both preCol's (i.e. D and P) from *M. edulis* are characterized by a common basic structure: a central collagen helix which is flanked by different flanking regions, which are each terminated by a histidine and DOPA rich terminus (see Figure 1).

The mechanism for the assembly of byssus collagens into fibers has been an elusive aspect of the byssus biochemistry. It is well recognized that the collagens undergo stabilization via cross-linking; however the chemistry is still not well understood. There are two distinct cross-linking possibilities: metal complexation and covalent bond formation between collagen units [8, 9]. Metal complexation is suggested by the high levels of iron, copper, nickel and zinc found in byssus and by the occurrence of metal-binding histidine-rich sequences in both terminals of the byssal proteins. Moreover, DOPA is present in both the termini of all Pre-Col's. Peptidyl-DOPA provides excellent metal binding sites and peptidyl-DOPA-Fe(III) chelates have been reported in the marine adhesive plaque mefp-1 [10]. Further, it has been shown that removal of metal ions from byssal fiber by EDTA reduces the yield strength of the fiber. Covalent cross-links have also been observed. They are generally formed by oxidative coupling between tyrosines, DOPA and cysteines. In a study of byssus stressed by conditions of high flow and aeration, the primary product of oxidation was found to be 5,5'-diDOPA[11]. Other possible coupling products like the Michael-type addition of lysines to oxidized DOPA have not been found [7].

Like "normal" collagen, each mussel collagen has a signal sequence of 20 amino acids which make sure that the alpha-chains are transported into the endoplasmatic reticulum. There, three identical alpha-chains assemble to a homotrimer. The ColD alpha-chain, which means the pepsin-cleaved preColD, has a molecular mass of 60kDa by SDS-PAGE and 47 kDa by MALDI-TOF mass spectometry (QIN et al., 1997). The alpha-chain of ColP, which means the pepsin-cleaved preColP, has a molecular mass of 55 kDa (by SDS-PAGE) and 40 kDa (MALDI-TOF), respectively (COYNE et al., 1997). The precursors of the alpha-chain are named preColD and preColP and have molecular masses of 95 and 97 kDa by means of SDS-PAGE analysis and 75 and 80 kDa respectively by analysis with MALDI-TOF mass spectometry (COYNE et al., 1997; QIN et al., 1997). Both collagens have characteristics which are typical for collagen type I-III. Both have an amount of more than 34 % of glycine and show a proline and hydroxyproline content of combined 20% within the collagen domain.

The flanking regions fully correspond to other structural proteins, namely elastin (preColP) and silk-fibroin (preColD). This structural construction gives an explanation for the mechanical behavior of mussel byssi. For this reason, it would be highly relevant to recombinantly produce the underlying mussel byssus collagens in order to use these extraordinary natural materials as building blocks in new technological applications.

OLSEN D ET AL ("Recombinant collagen and gelatin for drug delivery" ADVANCED DRUG DELIVERY REVIEWS, AMSTERDAM, NL, vol. 55, no. 12, 28 November 2003 (2003-11-28), pages 1547-1567) and BAEZ JULIO ET AL ("Recombinant microbial systems for the production of human collagen and gelatin" APPLIED MICROBIOLOGY AND BIOTECHNOLOGY, vol. 69, no. 3, December 2005, pages 245-252) disclose yeast cells for producing recombinant human collagen.

DEMING T J ET AL ("MUSSEL BYSSUS AND BIOMOLECULAR MATERIALS", CURRENT OPINION IN CHEMICAL BIOLOGY, CURRENT BIOLOGY LTD, LONDON, vol. 3, 1 january 1999 (1999-01-01), pages 100-105) and GOSLINE J ET AL ("ELASTIC PROTEINS: BIOLOGICAL ROLES AND MECHANICAL PROPERTIES", PHILOSOPHICAL TRANSACTIONS. ROYAL SOCIETY OF LONDON.BIOLOGICAL SCIENCES, ROYAL SOCIETY, LONDON, GB LNKD-DOI:10.1098/RSTB.2001.1022, vol. 357, no. 1418, 28 February 2002 (2002-02-28), pages 121-132) describe the use of mussel byssus proteins in the field of biotechnology or medicine.

The development of materials having defined characteristics, in particular of materials which are capable of regenerate themselves following stress or overloading has been of high interest in the material sciences for a long time. Composite structures are of gaining interest in technology, in particular for electronic components and devices, energy converters and other materials. By combination of materials having different mechanical characteristics, structural interfaces will be formed causing new technological problems.

Thus, for many applications it would highly desirable to provide a graduated structure thereby reducing the overall load of the material.

Furthermore, the use and application of mussel collagens in medicine is of great interest because of the high potential biocompatibility. Based on this, medical transplants and tissues could be generated having a high degree of immunocompatibility. The production of recombinant mussel collagens is an interesting and important technical problem which has to be solved before technical applications of mussel collagens may be envisioned.

Therefore it is an object underlying the present invention to provide recombinant mussel byssus proteins having enhanced characteristics as, in particular, improved capability of being expressed in high yield and good strength and flexibility. It is a further object of the present invention to provide recombinant mussel byssus proteins which can be specifically adapted to the required application by specific arrangement of the building blocks on which they are based to provide a graduated structure. Furthermore, it is an object of the present invention to provide expression vectors coding for recombinant mussel byssus proteins, which can be conveniently expressed in already known eucaryotic expression systems. Additionally, it is an object of the present invention to provide improved paper, textile and leather products. Additional objects are to provide new proteins and further materials based on recombinant mussel byssus proteins such as spheres, nanofibrils, hydrogels, threads, foams, films for use in biotechnology, medicine, pharmaceutical and food applications, cosmetics, in electronic devices and for other commercial purposes. It is a still further object of the present invention to provide a host cell, which is capable of expressing collagen like proteins, in particular mussel byssus proteins, in high yield and quality.

These objects are solved by the subject-matter of the independent claims. Preferred embodiments are set forth in the dependent claims.

Up to now, the expression of recombinant mussel byssus proteins has never been shown. This might be at least partially due to the complex process of expressing those proteins and threads made therefrom. The complexity of the biosynthesis of collagen leads to a reduced predictability of the outcome of any attempt to express recombinant collagens and, therefore, these attempts might presumably lead to improperly folded proteins, low yield or, in the worst case, to no expression of collagen at all.

In the present invention, a host cell system is provided which results in high yields of properly folded collagen like proteins, in particular of mussel byssus proteins.

The present invention in particular is directed to the following aspects and embodiments:

According to a first aspect, the present invention provides a yeast cell for producing a recombinant collagen like protein, which yeast cell has been transformed with the following elements:
a) a first expression vector which codes for said recombinant collagen like protein; and
b) a second expression vector comprising a nucleic acid coding for prolyl-4-hydroxylase (P4H);
wherein the recombinant collagen like protein is a recombinant mussel byssus protein comprising or consisting of one or more fragments of a mussel collagen domain flanked by a mussel elastin or mussel silk fibroin domain.

Due to the complexity of the biosynthesis of collagen, for the recombinant synthesis of collagen-like proteins, the inventors found out that some factors have to be considered, the most important one being the posttranslational modification in the endoplasmatic reticulum (ER) of proline to hydroxyproline by prolyl-4 hydroxylase, a tetrameric enzyme, which is composed of the two sub-units of alpha-PH ( = P4HA) and PDI ( = P4HB) (BULLEID et al., 2000). For this reason, procaryotic expression systems, for example bacterial expression systems, may not be used in the present invention.

Yeasts on the one hand offer the cell compartimentation which is required for the synthesis of collagen, on the other hand, however, they are lacking the enzyme prolyl-hydroxylase (P4H) which is required for the synthesis of collagen. Apart therefrom, yeasts would be a desirable expression system for recombinant collagens since their cultivation, also in large scale expression systems, is comparably easy to achieve and the yield of recombinant protein therefrom is superior to other expression systems. Thus, expression in yeast might lead to an efficient (and also cost-effective) production of recombinant collagen like proteins, in particular of mussel byssus proteins. However, as a result of the above drawbacks of yeast cells, an expression of those proteins in yeast cells has not been achieved up to now.

It could be shown by the inventors for yeast cells, which do not possess P4H, that human P4H subunits can be produced recombinantly and can be correctly folded. Apart therefrom, it could be shown for these yeast strains that by co-expression of both human P4H subunits, the synthesis of mussel byssus collagen is possible and folded, stabile collagen is formed.

Interestingly, the co-expression of the genes of both P4H subunits is sufficient for the formation of a stable triple helix in yeast and no further enzymes or folding promoters or chaperones specific for collagen are required, as for example Hsp47, or in other words, the chaperones which are inherent to yeast are sufficiently "active". Human collagens, recombinantly produced in yeast possessed the same content of hydroxyproline and, furthermore, are identical in respect to many other characteristics compared to native collagens.

By efficient transport in the ER of yeast, signal sequences of the co-expressed P4H subunits play an important role. A maximum sufficiency of localization can be achieved by replacing in a preferred embodiment, the human with a yeast signal sequence, for example from the S. *cerevisae* pheromone *mating factor alpha1* (MFa). The P4H subunits modified by the MFa signal sequence were effectively transported into the lumen of the ER.

More preferably, the signal sequence is mating factor alpha 1 (MFa) of *S. cerevisiae* according to SEQ ID NO: 10.

As a yeast cell, preferably *S. cerevisiae, Schizosaccharomyces pombe, Pichia pastoris, Candida albicans,* or *Hansenula polymorpha* cells might be used.

The first expression vector preferably further comprises one or more regulatory elements. The expression vector must be suitable for expression in yeast cells.

Preferably, the regulatory elements contain a promoter selected from constitutive or inducible promoters, more specifically from GPD, GAL4, CUP1, MET25, GAL1 or GAL1-10.

In a further embodiment, the expression vector is a plasmid.

This recombinant mussel byssus protein is composed of one or more types of building blocks, which provide different characteristics to the protein formed: as mentioned above, elastin and silk-fibroin have certain mechanical characteristics, which can give an explanation for the mechanical behavior of mussel byssi and, thus, also for the design of recombinantly produced mussel byssus proteins.

Therefore, these fragments can be used as one single type of fragment only, or, as an alternative, the recombinant protein can comprise two or more different fragments. For example, if great elasticity is wanted, the protein may only or predominantly comprise fragments of collagen flanked by elastin. If great stiffness and strength is required, the protein may comprise fragments of collagen flanked by silk-fibroin. As a further and preferred alternative, the protein may comprise a mixture of both types of fragments, for example forming a gradient from one region to the other. Thus, a protein/thread can be formed having specifically adapted configurations, i.e. parts having higher elasticity and parts having higher stiffness etc.

The term "flanked" means that elastin (or silk-fibroin) is present on both sides of the collagen domain.

The above fragments may be naturally derived, for example, the fragments may be obtained from *Mytilus sp.,* preferably from *M. edulis, M. galloprovincialis, M. californians,* or *Geukeria demissa.*

According to a preferred embodiment, the recombinant mussel byssus protein of the invention comprises or consists of one or more of the fragments preColP and/or preColD or variants thereof. These fragments have been outlined above. Both preCol's (i.e. D and P) are derived from *M. edulis* and are characterized by a common basic structure: a central collagen helix which is flanked by different flanking regions, which are each terminated by a histidine and DOPA rich terminus (see Figure 1). The flanking regions fully correspond to known structural proteins, namely elastin (preColP) and silk-fibroin (preColD).

The sequences of preColP and preColD are translated from the respective nucleic acids. Therefore, whenever amino acids are recited herein in the following, they are referring to preColP and preColD and these sequences will be used in the various technological applications mentioned hereinabove. The nucleic acid sequences mentioned herein in the first place are directed to preColP and preColD encoding sequences.

According to a further embodiment, the recombinant protein of the invention comprises or consists of one or more fragments of SEQ ID NO: 3 and/or 4 or variants thereof. The Seq ID No's reflect the sequences of preColP and preColD.

As mentioned above, the present invention also comprises variants of those amino acid sequences. For example, said variants may contain one or more substitutions, insertions and/or deletions when compared to the amino acid sequences mentioned above.

In particular variants of the protein, for example deletions, insertions and/or substitutions in the sequence, which cause so-called "silent" changes, are considered to be part of the invention.

Preferably are such amino acid substitutions the result of substitutions which substitute one amino acid with a similar amino acid with similar structural and/or chemical properties, i.e. conservative amino acid substitutions.

Amino acid substitutions can be performed on the basis of similarity in polarity, charges, solubility, hydrophobic, hydrophilic, and/or amphipathic (amphiphil) nature of the involved residues. Examples for hydrophobic amino acids are alanine, leucine, isoleucine, valine, proline, phenylalanine, tryptophan and methionine. Polar, neutral amino acids include glycine, serine, threonine, cysteine, thyrosine, asparagine and glutamine. Positively (basic) charged amino acids include arginine, lysine and histidine. And negatively charged amino acids include aspartic acid and glutamic acid.

"Insertions" or "deletions" usually range from one to five amino acids. The allowed degree of variation can be experimentally determined via methodically applied insertions, deletions or substitutions of amino acids in a polypeptide molecule using recombinant DNA methods. The resulting variants can be tested for their characteristics, in particular their mechanical characteristics.

It is noted that the term "variant" as used herein also comprises the above amino acid sequences of preColP and preColD, wherein the first 19 amino acids constituting the original mussel signal sequence were replaced by other signal sequences. A preferred example hereof is replacement of the mussel signal sequence by signal sequence alpha MF (SEQ ID NO: 10: "MRFPSIFTAV LFAASSALA"). This signal sequence in particular is suitable for expression of the nucleic acids in yeasts.

The present invention also provides an isolated nucleic acid encoding the recombinant protein as defined above. The term "isolated" as used herein with reference to nucleic acids refers to a naturally-occurring nucleic acid that is not immediately contiguous with both of the sequences with which it is immediately contiguous (one on the 5'end and one on the 3'end) in the naturally-occurring genome of the organism from which it is derived.

For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e. g., a cDNA or a genomic DNA fragment produced by PCR or restriction endonuclease treatment) independent of other sequences as well as recombinant DNA that is incorporated into a vector, an autonomously replicating plasmid, a virus (e. g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" also includes any non-naturally-occurring nucleic acid since non-naturally-occurring nucleic acid sequences are not found in nature and do not have immediately contiguous sequences in a naturally-occurring genome. For example, non-naturally-occurring nucleic acid such as an engineered nucleic acid is considered to be isolated nucleic acid. Engineered nucleic acid can be made using common molecular cloning or chemical nucleic acid synthesis techniques. Isolated non-naturally-occurring nucleic acids can be independent of other sequences, or incorporated into a vector, an autonomously replicating plasmid, a virus (e. g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

It will be apparent to those of skill in the art that a nucleic acid existing among hundreds to millions of other nucleic acid molecules within, for example, cDNA or genomic libraries, or gel slices containing a genomic DNA restriction digest is not to be considered an isolated nucleic acid.

A nucleic acid encoding the above amino acids may be a nucleic acid sequence coding for the mature or the immature amino acid sequence of the recombinant mussel byssus protein.

In a preferred embodiment, the isolated nucleic acid comprises or consists of the nucleic acid of SEQ ID NO: 1 and/or 2 or variants thereof. These variants are each defined as having one or more substitutions, insertions and/or deletions as compared to the sequences of SEQ ID NO: 1 or 2, provided that said variants hybridize under moderately stringent or stringent conditions to a nucleic acid which comprises the sequence of SEQ ID NO: 1 or 2, or provided that said variants comprise nucleic acid changes due to the degeneracy of the genetic code, which code for the same or a functionally equivalent amino acid as the nucleic acid sequence of SEQ ID NO: 1 or 2.

As mentioned above, the present invention also encompasses a variant of said nucleic acids, wherein the nucleic acids coding for the first 19 amino acids (signal sequence) were replaced, preferably by the yeast signal sequence MFa (SEQ ID NO: 10).

Stringency of hybridization, as used herein, refers to conditions under which polynucleotide duplexes are stable. As known to those of skill in the art, the stability of duplex is a function of sodium ion concentration and temperature (see, for example, Sambrook et al., Molecular Cloning: A Laboratory Manual 2nd Ed. (Cold Spring Harbor Laboratory, (1989)). Stringency levels used to hybridize can be readily varied by those of skill in the art.

Stringent washing conditions mean 0.2 x SSC (0.03 M NaCl, 0.003 M sodium citrate, pH 7)/ 0.1% SDS at 65°C. For shorter fragments, e.g. oligonucleotides up to 30 nucleotides, the hybridization temperature is below 65°C, for example at 50°C, preferably above 55°C, but below 65°C. Stringent hybridization temperatures are dependent on the size or length, respectively of the nucleic acid and their nucleic acid composition and will be experimentally determined by the skilled artisan. Moderate stringent hybridization temperatures are for example 42°C und washing conditions with 0.2 x SSC/0.1% SDS at 42°C.

The P4H used in the present invention preferably is human or mussel P4H.

In a second aspect, a kit of parts or a co-expression system for use in the production of a recombinant mussel byssus protein comprising the following constituents is provided:
a) the first expression vector as defined herein; and
b) the second expression vector as defined above.

This kit of parts or co-expression system may be efficiently used in expressing the recombinant mussel byssus protein in yeast cells.

In a still further aspect, a method of producing a recombinant mussel byssus protein is disclosed comprising the steps of:
a) providing a yeast cell as defined hereinabove;
b) transforming said yeast cell with a first and second expression vector as explained above or with the co-expression system as explained above;
c) expressing the recombinant mussel byssus protein from said yeast cell under suitable conditions; and
d) recovering said recombinant protein.

Furthermore, a method for producing threads from a recombinant mussel byssus protein is provided, comprising the following steps:
a) providing a recombinant mussel byssus protein as produced in accordance with the above method, and
b) (electro)spinning or molding said protein into threads by a suitable method.

The spinning may preferably be done by electrospinning. Electrospinning is a fiber formation technique that uses electrostatic forces to create continuous, nanometer diameter fibers. A wide variety of natural and artificial polymers have been electrospun from the solution and melt phase and are of interest for an assortment of application areas that require high surface area materials (filtration membranes and biomedical devices).

Further disclosed herein but not encompassed by the invention is a protein or thread obtainable by one of the above methods.

The proteins/threads find application preferably in the field of biotechnology and/or medicine.

For example, they might be used for the manufacture of wound closure or coverage systems or suture materials. Furthermore, the proteins/threads may preferably be used for the manufacture of replacement materials, preferably artificial cartilage or tendon materials.

Additionally, the threads/proteins can be used in the manufacture of medical devices such as medical adhesive strips, skin grafts, replacement ligaments, and surgical mesh; and in a wide range of industrial and commercial products, such as clothing fabric, bullet-proof vest lining, container fabric, bag or purse straps, cable, rope, adhesive binding material, non-adhesive binding material, strapping material, automotive covers and parts, aircraft construction material, weatherproofing material, flexible partition material, sports equipment; and, in fact, in nearly any use of fiber or fabric for which high tensile strength and elasticity are desired characteristics. Adaptability and use of the stable fiber product in other forms, such as a dry spray coating, bead-like particles, or use in a mixture with other compositions is also possible.

It is explicitely noted that preferred applications of the mussel byssus collagens are in the manufacture and processing of clothing fabric (textiles) and leather, automotive covers and parts, aircraft construction materials as well as in the manufacture and processing of paper.

The recombinant mussel byssus proteins may be added to cellulose and keratin and collagen products. Further disclosed herein but not encompassed by the invention is a paper or a skin care and hair care product, comprising cellulose and/or keratin and/or collagen and the proteins described above. Papers and skin care and hair care products, in which the proteins described above are incorporated are showing improved characteristics, in particular improved tensile strength or tear strength.

Furthermore, the recombinant mussel byssus proteins may be used as a coating for textile and leather products, thereby conferring stability and durability to the coated product. The proteins in particular show applicability for coating leather products, since in this case, tanning and its negative effects for environment can be avoided or at least reduced.

Further disclosed herein but not encompassed by the invention are products containing said mussel byssus proteins, for example, wound closure or coverage systems, suture materials, replacement materials, preferably artificial cartilage or tendon materials, cosmetics, drug delivery vehicles, fabrics, textile, paper product, leather product, automotive parts or aircraft parts. Further disclosed herein but not encompassed by the invention are materials based on recombinant mussel byssus proteins such as spheres, nanofibrils, hydrogels, foams, films.

Unless otherwise defined, all technical and scientific terms used herein have the same meaning as commonly understood by one of ordinary skill in the art to which this invention pertains. In case of conflict, the present specification, including definitions, will control. In addition, the examples are illustrative only and not intended to be limiting.

The invention is now further illustrated by examples and the accompanying drawings, which are showing the following:
- Fig. 1: is illustrating the general structure of mussel byssus collagens;

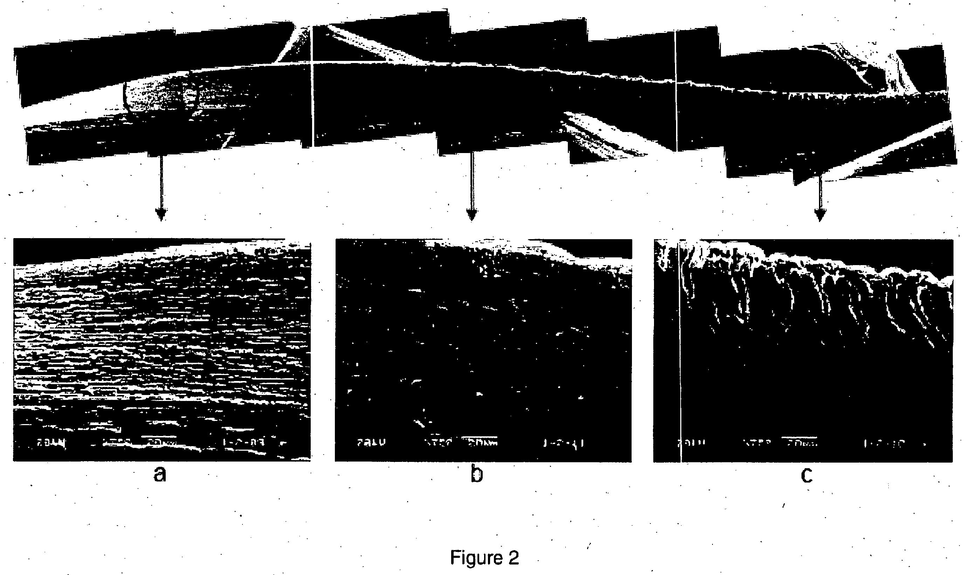

- Fig. 2: depicts a series of SEM images of byssus threads in direction distal to proximal - the marked portions are each enlarged below. a) distal; b) median; c) proximal;
- Fig. 3: shows the structure of mussel byssus;

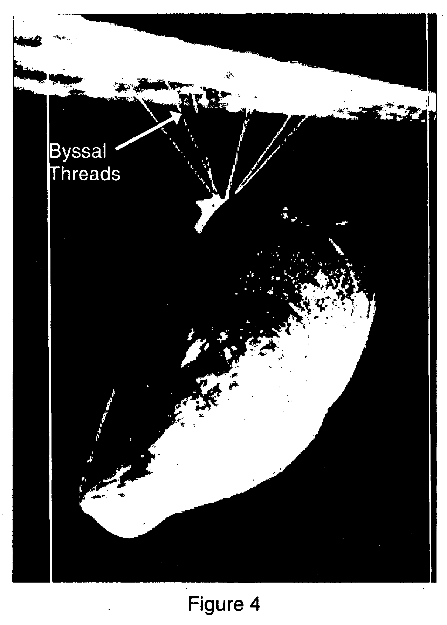

- Fig. 4: illustrates a mussel adhered to a solid surface by byssus threads;
- Fig. 5:: (A) Distribution of preCols in the thread. (B) Schematic of a collagenous subunit with flanking domains. Terminal regions denoted by diamonds are His-rich. DOPA is designated by Y. (C) Model of cross-linking interactions between axial and lateral preCols;
- Fig. 6:: Design of the P4H construct;
- Fig. 7:: Design of oligonucleotides to generate α-MF signal sequences ready to be cloned into respective expression plasmids;
- Fig. 8:: Cloning strategy for α-PH;

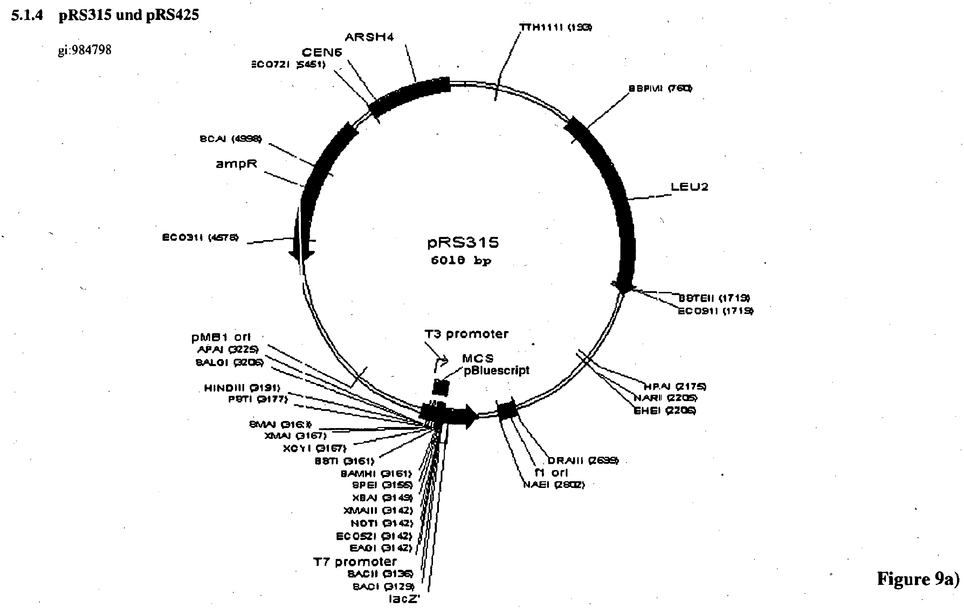

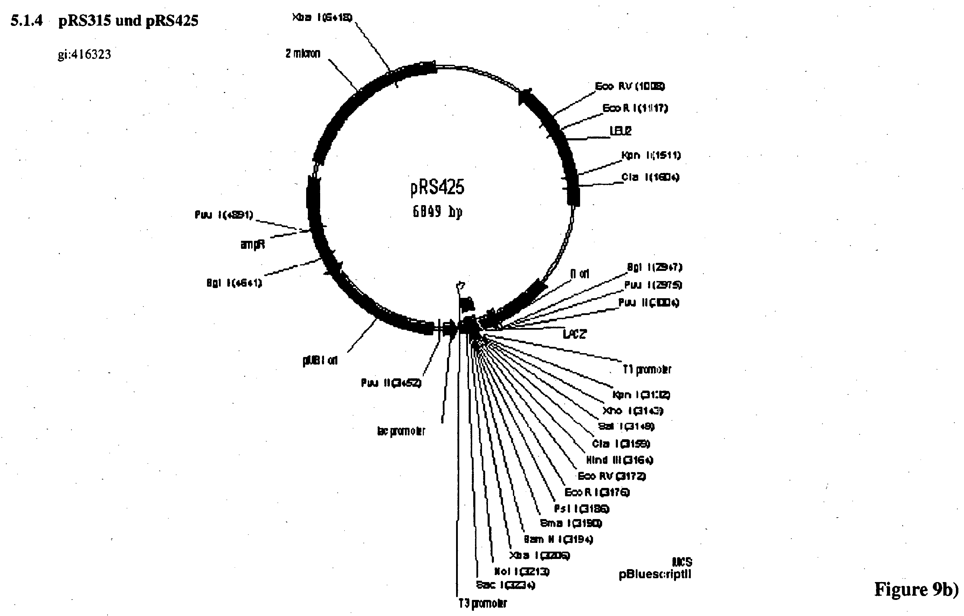

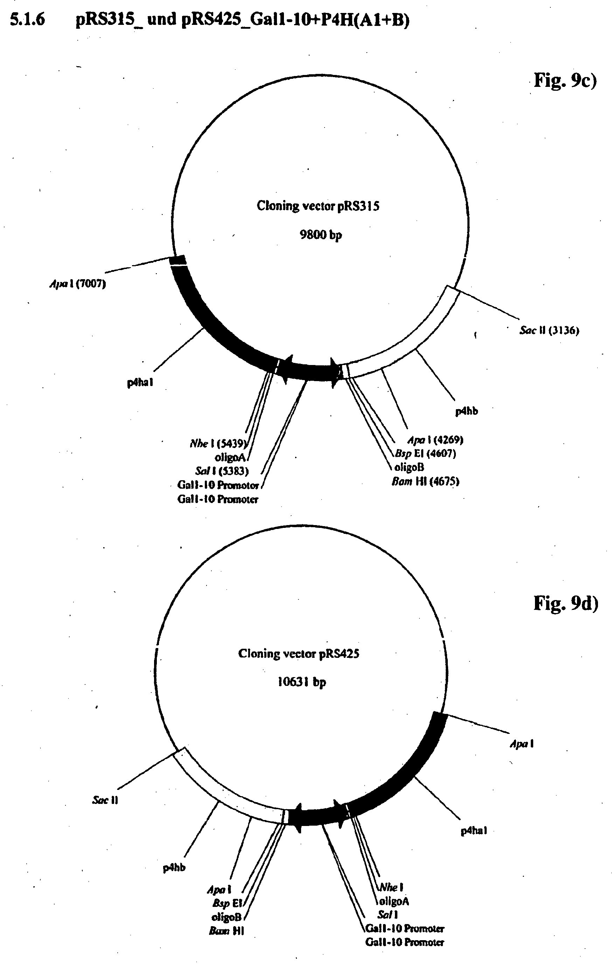

- Fig. 9:: Vector maps.

### Examples:

### Expression of Collagen proteins of Mussel Byssus in Yeast

Collagen synthesis in general reflects a complex biochemical process. The process requires e.g. post-translational modification of certain prolines of the respective collagens to 4-hydroxyproline in the ER by the enzyme Prolyl 4-hydroxylase (P4H). P4H, an α₂ß₂ tetramer in vertebrates, plays a central role in the synthesis of collagens. 4-hydroxyproline residues, generated by P4H, are essential for the folding of the newly synthesized collagen polypeptide chains into triple-helical collagen molecules [13].

### Human Prolyl-4-Hydroxylase expression construct

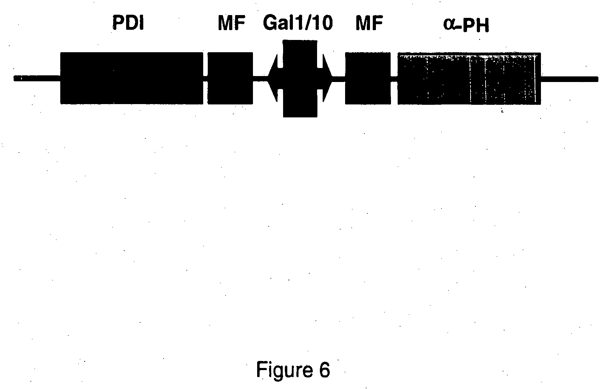

The construct of P4H requires the cloning of a signal sequence into the yeast vector adjacent to the genes for the two subunits of P4H, α-PH and PDI. Both genes are placed under the control of a bi-directional promotor, which is induced in the presence of Galactose (Gal 1/10) (see Figure 6). The signal sequence is required for translocation of P4H subunits into the ER, where they can assemble into the native tetramer. Maximum efficiency for localization has been achieved when the human signal sequence is replaced by yeast's own signal sequence of the mating factor α-MF [12]. See Figure 6 in this context.

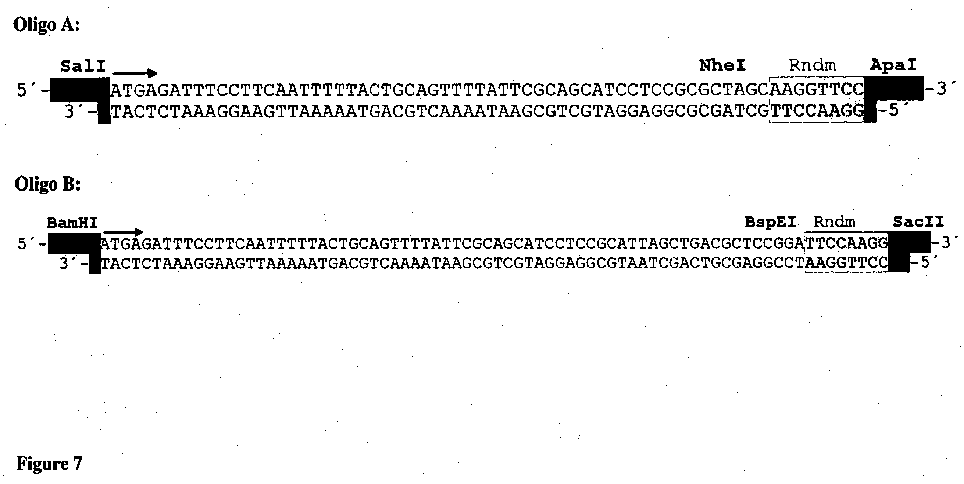

The gene for α-PH (without signal sequence) is amplified by PCR from a c-PNA library from HepG2 liver cells (provided by Professor Adamski, GSF Munich, Germany), while the c-DNA of the beta-subunit (PDI) (without signal sequence) will be amplified from an *E. coli* cloning vector (provided by Professor Neil Bulleid, University of Manchester, UK). For each gene a respective αMF signal sequence will be engineered based on two single stranded oligonucleotides. The oligos A and B are planned in a way (see figure 7) that after annealing the double stranded DNA can be directly cloned into respective vectors.

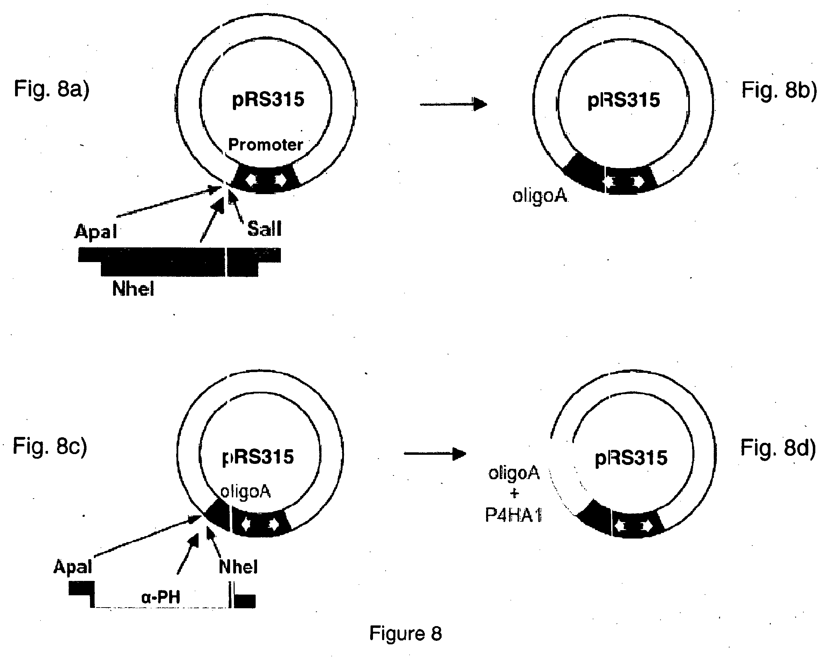

The cloning strategy for α-PH is shown as an example (Figure 8). Cloning of the cDNA of PDI will be performed in an identical way. Two different yeast vectors will be used: -pRS315 (CEN, reflecting a single copy number plasmid) und pRS425 (2µ, reflecting a multi copy plasmid), both containing the bi-directional Gal1/10 promotor, allowing the simultaneous expression of both subunits from one plasmid.

### Recombinant synthesis of preColD and preColP

Recombinant synthesis of preColD and preColP, two major protein components of Mussel Byssus is an example of the present invention. The c-DNA of PreColP and PreColD in *E. coli* cloning vectors has been obtained from Prof. Waite (UCSB, USA). The cDNA is amplified by PCR and cloned into different yeast expression vectors. The vectors differ in copy number per cell, as well as in the choice of the activator (either the constitutive promotor GPD or the inducible promotor GAL4). Also the original signal sequence will be replaced by the signal sequence of the yeast α-MF for maximum localization efficiency.

### Detection of ColP and ColD during recombinant synthesis

The test for the efficient recombinant synthesis of mussel collagen requires availability of polyclonal antibodies against mussel collagen. Preliminary tests with polyclonal antibodies against human collagen type I-III showed a very weak cross-reactivity against chemically denatured collagen from mussel byssus. This cross-reactivity is not sufficient to detect the levels of collagen present during the recombinant synthesis. Hence antibodies need to be raised against mussel collagen. In order to be able to raise antibodies, purified native mussel collagen is required. Byssus will be extracted from fresh mussels and purified using several chromatographic methods (reverse phase chromatography among others).

The purified protein samples, which contain both preColD and preColP, are used to immunize rabbits and generate antibodies.

### Biophysical studies on recombinant Collagen

Various physical methods can be used to characterize the individual proteins preColP/preColD and to evaluate the efficiency of fiber formation on self-assembly. These methods include far- and near-UV circular dichroism (CD), static and dynamic light scattering, fourier transformed infrared spectroscopy (FTIR), electron microscopy (EM), atomic force microscopy (AFM) and field flow fractionation (FFF).

### Characterization of individual preColP andpre ColD

CD and FTIR will be used to determine the secondary and tertiary structures of preColP and preColD. Their chemical and thermal stability will also be tested under various conditions. Data on the shape of the proteins involved in the collagen formation are provided by light scattering, by AFM and TEM.

### Evaluation of rate and efficiency of fiber formation

Secondary and tertiary structure of mussel byssus collagen are analyzed by CD and FTIR. AFM and EM will provide information on the quaternary structure and morphology of the assembled aggregates and fibers. FFF, a one-phase matrix-free chromatography, would be used to evaluate the different kinds of species formed during assembly of the collagen. Since FFF is a matrix-free chromatography technique, it can separate different dissolved macromolecules, especially fibers, which can not be separated by other classical chromatographic techniques.

Kinetics of the assembly process will also be investigated with CD and FTIR, which can be performed as a function of time by monitoring changes in the secondary and tertiary structure during the fiber formation. Further, static light scattering, dynamic light scattering and time-lapse AFM allow to monitor protein assembly in real-time.

Fluorescent dyes can also be used to investigate the structural changes associated with protein assembly. The fluorescent properties of some dyes, such as the N-benzyl derivatives of 3-chloro-6-methoxy-9 aminoacridine and amino naphthalene sulfonic acids, change with the polarity of the protein environment. Therefore, labeling of collagen with these dyes are used to study its assembly process.

### Study of the role of metals in the assembly and cross-linking of Collagens

### Role of GGH in DOPA and tyrosine cross-links

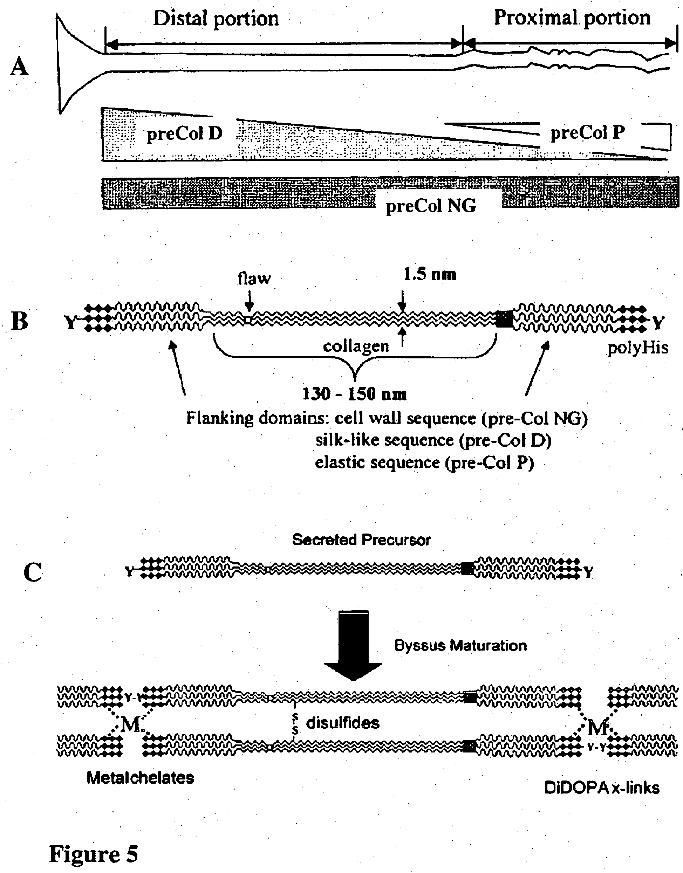

The amino acid sequence GGH has been observed at the carboxylterminus of both preColP and preColD. The tripeptide NH2-Gly-Gly-His-COOH(GGH) mediates cross-linking of associated proteins in solution in the presence of nickel acetate [Ni(OAc)2] and oxidant magnesium monoperoxyphthalate (MMPP) [18, 19]. Further, the peptide provides a favorable coordination environment for the nickel center, and a putative Ni(III) intermediate is thought to abstract an electron from the aromatic ring of an accessible tyrosine, leading to a tyrosyl radical after the loss of a proton (see figure 5). The highly activated radical intermediate couples to a nearby tyrosine leading to a cross-linked adduct.

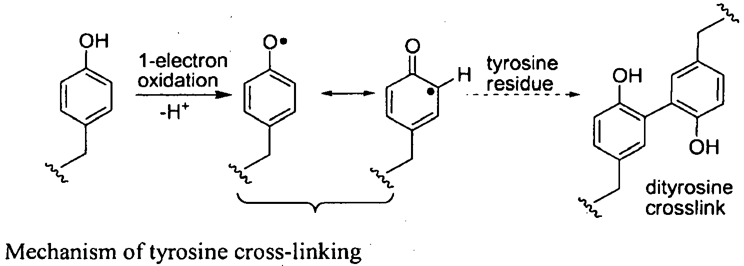

A possible role for the GGH in the carboxylterminus of mussel collagens could be to bind Ni(II) in order to form the active catalyst Ni-GGH. This complex can slowly catalyze aerial oxidation of tyrosine and DOPA to form cross-links. The proximity of the catalyst to tyrosine and/or DOPA would significantly increase the oxidation rates. To test this hypothesis, the GGH sequence could be genetically deleted or modified so that it would not bind nickel. The rate of cross-linking and assembly would be monitored by methods described above.

### Chemical oxidation of tyrosines to form cross-links in Collagen

Visible-light irradiation in the presence of ruthenium(II)tris(bipyridyl) dication [Ru(bpy)₃²⁺] and an electron acceptor such as ammonium persulfate (APS) [18, 20, 21] induces very efficient cross-linking between contacting proteins. This process is highly efficient and the mechanism has been assumed to be similar to that of Ni/GGH/MMPP. The fiber formed from self-assembly of preColP and / or preColD will be subject to irradiation with [Ru(bpy)₃²⁺] in the presence of APS. This should lead to increased cross-linking of tyrosine/DOPA in byssal collagen and lead to fibers with altered mechanical properties, which will be assessed upon physico-chemical characterization as described above.

Further examples and sequences:
DNA sequences of mussel collagen preColP and proColD are provided in the following. The cDNA of both preCol proteins (P and D) were integrated in the pGEM-T cloning vectors. In order to verify the starting material, both cDNAs were completely sequenced and as standard primers T7 and SP6 were used and as internal primers preCol (P or D)-T7/1 and SP6/1, respectively, were used. The obtained DNA sequences showed differences as regards the published versions of both preCols and were compared accordingly.

### preColP (SEQ ID NO:1)

Due to the degeneration of the genetic code, not every base exchange is leading to an amino acid exchange. Therefore, the DNA sequences were translated into the amino acid sequences and were compared. Here, the alignment of the published sequence (COYNE et al., 1997) [gi:2386675] (variant P38, (COYNE & WAITE, 2000)) with the sequence of preColP obtained by sequencing is shown. The database sequence corresponds to SEQ ID NO: 9, the sequenced preColP sequence is SEQ ID NO:3.

COYNE & WAITE already showed the existence of different preColP variants (P22, P33 and P38) in certain partial regions of their cDNA sequence (COYNE & WAITE, 2000). If these short, known sequence regions of variant P22 are compared with the present DNA sequence of preColP, a matching of 100% is achieved.

### preColD (SEQ ID NO:2)

In the following, the alignment of the published sequence (QIN et al., 1997) [gi:2772914] with the overall sequence obtained by sequencing of preColD is shown. The DNA sequences were translated into the protein sequence. It is noted that the database sequence is SEQ ID NO: 8 and the sequence obtained by sequencing is SEQ ID NO: 4.

There are significant differences in both sequences: The preColD sequence used is by 250 amino acids shorter than the published sequence. The major part of the amino acids in the collagen domain is missing. Therefore, the presently disclosed preColD gene is an up to now unpublished and unknown version of the preColD gene. It is noted that the truncation of the collagen domain increases the amount of silk fibroin domains in the whole protein and therefore, the behavior of the overall protein will be different.

### EXPRESSION CONSTRUCT of P4H

In the following, the DNA sequence after expression plasmid for P4H in the region of *SacII* to *ApaI* is shown as double strand. The beginning and the end of MFa/P4H fusion constructs are both printed. The used restriction sites are underligned.

### SEQ ID NO: 7

### Protein sequences

### MFa - P4HA (SEQ ID NO:5)

### MFa - P4HB (SEQ ID NO:6)

### Sequence of MFa (SEQ ID NO: 10)

MRFPSIFTAV LFAASSALA

### References:

1. Yonge, M. (1962). On the significance of the byssus in the bivalvia and its effects in evolution. J. Mar. Biol. Ass. U. K. 42, 113-125.
2. Qin, X.-X., Coyne, K.J., and Waite, J.H. (1997). Tough tendons. Mussel byssus has collagen with silk-like domains. Journal of Biological Chemistry 272, 32623-32627.
3. Waite, J.H. (1992). Results Probl. Cell Differ. 19, 27.
4. Qin, X.-X., and Waite, J.H. (1995). Exotic Collagen Gradients in the Byssus of ht eMussel Mytilus Edulia. The Journal of Experimental Biology 198, 633-644.
5. Vaccaro, E., and Waite, J.H. (2001). Yield and Post-Yield Behavior of Mussel Byssal Thread: A Self-Healing Biomolecular Material. Biomacromolecules 2, 906-911.
6. Coyne, K.J., Qin, X.-X., and Waite, J.H. (1997). Extensible collagen in mussel byssus: a natural block copolymer. Science (Washington, D. C.) 277, 1830-1832.
7. Waite, J.H., Qin, X.-X., and Coyne, K.J. (1998). The peculiar collagens of mussel byssus. Matrix Biology 17, 93-106.
8. Coombs, T.L., and Keller, P.J. (1981). Mytilus byssal threads as an environmental marker for metal ions. Aquat. Toxicol. 1981, 291-300.
9. Swann, C.P., Adewole, T., and Waite, J.H. (1998). Preferential manganese accumulation in dreissenid byssal threads. Comparative Biochemistry and Physiology, Part B: Biochemistry & Molecular Biology 119B, 755-759.
10. Taylor, S.W., Chase, D.B., Emptage, M.H., Nelson, M.J., and Waite, J.H. (1996). Ferric Ion Complexes of a DOPA-Containing Adhesive Protein from Mytilus edulis. Inorganic Chemistry 35, 7572-7577.
11. Sun, C., Vaccaro, E., and Waite, J.H. (2001). Oxidative stress and the mechanical properties of naturally occurring chimeric collagen-containing fibers. Biophysical Journal 81, 3590-3595.
12. Myllyharju, J., Nokelainen, M., Vuorela, A., and Kivirikko, K.I. (2000). Expression of recombinant human I-III collagens in the yeast Pichia pastoris. Biochem. Soc. Trans. 28, 353-357.
13. Prockop, D.J., and Kivirikko, K.I. (1995). Annu. Rev. Biochem. 64, 403-434.
14. Olsen, D.R., Leigh, S.D., Chang, R., McMullin, H., Ong, W., Ernest, T., Chisholm, G., Birk, D.E., Berg, R.A., Hitzeman, R.A., and Toman, P.D. (2001). Production of Human Type 1 Collagen in Yeast Reveals Unexpected New Insights into Molecular assembly of Collagen Trimers. J. Biol. Chem. 276, 24038-24043.
15. Scheibel, T. (2004). Spider silks: recombinant synthesis, assembly, spinning, and engineering of synthetic proteins. Microbial Cell Factories 3, No pp. given.
16. Huemmerich, D., Scheibel, T., Vollrath, F., Cohen, S., Gat, U., and Ittah, S. (2004). Novel Assembly Properties of Recombinant Spider Dragline Silk Proteins. Current Biology 14, 2070-2074.
17. Huemmerich, D., Helsen, C.W., Quedzuweit, S., Oschmann, J., Rudolph, R., and Scheibel, T. (2004). Primary Structure Elements of Spider Dragline Silks and Their Contribution to Protein Solubility. Biochemistry 43, 13604-13612.
18. Brown, K.C., and Kodadek, T. (2001). Protein cross-linking mediated by metal ion complexes. Metal Ions in Biological Systems 38, 351-384.
19. Fancy, D.A., Denison, C., Kim, K., Xie, Y., Holdeman, T., Amini, F., and Kodadek, T. (2000). Scope, limitations and mechanistic aspects of the photo-induced cross-linking of proteins by water-soluble metal complexes. Chemistry & Biology 7, 697-708.
20. Burdine, L., Gillette, T.G., Lin, H.-J., and Kodadek, T. (2004). Periodate-Triggered Cross-Linking of DOPA-Containing Peptide-Protein Complexes. Journal of the American Chemical Society 126, 11442-11443.
21. Kim, K., Fancy, D.A., Carney, D., and Kodadek, T. (1999). Photoinduced Protein Cross-Linking Mediated by Palladium Porphyrins. Journal of the American Chemical Society 121, 11896-11897.

Further references:
Brake, A.J. (1990) Alpha-factor leader-directed secretion of heterologous proteins from yeast. Methods Enzymol. 185: 408-21
Bulleid, N.J., John, D.C. & Kadler, K.E. (2000) Recombinant expression systems for the production of collagen. Biochem. Soc. Trans. 28: 350-3
Coyne, K.J. & Waite, J.H. (2000) In search of molecular dovetails in mussel byssus: from the threads to the stem. J. Exp. Biol. 203: 1425-31
Keizer-Gunnink, I., Vuorela, A., Myllyharju, J., Pihlajaniemi, T., Kivirikko, K.I. & Veenhuis, M. (2000) Accumulation of properly folded human type III procollagen molecules in specific intracellular membranous compartments in the yeast Pichia pastoris. Matrix Biol. 19: 29-36
Lucas, J.M., Vaccaro, E. & Waite, J.H. (2002) A molecular, morphometric and mechanical comparison of the structural elements of byssus from Mytilus edulis and Mytilus galloprovincialis. J. Exp. Biol. 205: 1807-1
Mascolo, J.M. & Waite, J.H. (1986) Protein gradients in byssal threads of some marine bivalve molluscs. J. Exp. Zool. 240: 1-7
Qin, X.X. & Waite, J.H. (1998) A potential mediator of collagenous block copolymer gradients in mussel byssal threads. Proc. Natl. Acad. Sci. USA 95: 10517-22
Sikorski R.S. & Hieter P. (1989) A system of shuttle vectors and yeast host strains designed for efficient manipulation of DNA in Saccharomyces cerevisiae. Genetics 122, 19-27
Toman, P.D., Chisholm, G., McMullin, H., Giere, L.M., Olsen, D.R., Kovach, R.J., Leigh, S.D., Fong, B.E., Chang, R., Daniels, G.A., Berg, R.A. & Hitzeman, R.A. (2000) Production of recombinant human type I procollagen trimers using a four-gene expression system in the yeast Saccharomyces cerevisiae. J. Biol. Chem. 275: 23303-9
Vaughn, P.R., Galanis, M., Richards, K.M., Tebb, T.A., Ramshaw, J.A. & Werkmeister, J.A. (1998) Production of recombinant hydroxylated human type III collagen fragment in Saccharomyces cerevisiae. DNA Cell Biol. 17: 511-8
Vuorela, A., Myllyharju, J., Nissi, R., Pihlajaniemi, T. & Kivirikko, K.I. (1997) Assembly of human prolyl 4-hydroxylase and type III collagen in the yeast pichia pastoris: formation of a stable enzyme tetramer requires coexpression with collagen and assembly of a stable collagen requires coexpression with prolyl 4-hydroxylase. EMBO J. 16: 6702-12
Waite, J.H., Vaccaro, E., Sun, C. & Lucas, J.M. (2002) Elastomeric gradients: a hedge against stress concentration in marine holdfasts? Philos. Trans. R. Soc. Lond B Biol. Sci. 357: 143-53

## Claims

1. A yeast cell for producing a recombinant collagen like protein, which yeast cell has been transformed with the following elements:
a) a first expression vector which codes for said recombinant collagen like protein; and
b) a second expression vector comprising a nucleic acid coding for prolyl-4-hydroxylase (P4H),
wherein the recombinant collagen like protein is a recombinant mussel byssus protein comprising or consisting of one or more fragments of a mussel collagen domain flanked by a mussel elastin or mussel silk fibroin domain.

2. The yeast cell of claim 1, wherein said P4H sequence is linked to a signal sequence for efficient transport of said sequence to the ER of said yeast cell, wherein the signal sequence preferably is mating factor alpha 1 (MFa) of *S. cerevisiae.*

3. The yeast cell of claims 1 or 2, wherein the yeast cell is a *S. cerevisiae, Schizosaccharomyces pombe, Pichia pastoris, Candida albicans,* or *Hansenula polymorpha* cell.

4. The yeast cell of one or more of claims 1-3, wherein the first expression vector further comprises one or more regulatory elements, wherein the regulatory elements preferably contain a promoter selected from constitutive or inducible promoters.

5. The yeast cell of claim 4, wherein the promoter is selected from GPD, GAL4, CUP1, MET25, GAL1 or GAL1-10.

6. The yeast cell of one or more of the preceding claims, wherein the expression vectors are plasmids.

7. The yeast cell of one or more of the preceding claims, wherein the fragments are derived from *Mytilus sp.,* preferably *M*. *edulis, M. galloprovincialis, M. californians,* or *Geukeria demissa,* or wherein the recombinant mussel byssus protein comprises or consists of one or more of the fragments preColP and/or preColD or variants thereof.

8. The yeast cell of one or more of the preceding claims, wherein the recombinant protein comprises or consists of the amino acid sequence of SEQ ID NO: 3 and/or 4 or variants thereof.

9. The yeast cell of one or more of the preceding claims, wherein in the recombinante protein the signal sequence of the respective amino acid sequence is replaced by a yeast specific signal sequence, preferably by mating factor alpha 1 (MFa) of S. *cerevisiae,* or wherein the P4H is human or mussel P4H.

10. A kit of parts or a co-expression system for use in the production of a recombinant mussel byssus protein comprising the following constituents:
a) the first expression vector as defined in one or more of claims 1-9; and
b) the second expression vector as defined in one or more of claims 1-9.

11. A method of producing a recombinant mussel byssus protein comprising the steps of:
a) providing a yeast cell;
b) transforming said yeast cell with a first and second expression vector as defined in one or more of claims 1-9 or with the co-expression system of claim 10;
c) expressing the recombinant mussel byssus protein from said yeast cell under suitable conditions; and
d) recovering said recombinant protein.

12. A method for producing threads from a recombinant mussel byssus protein comprising the following steps:
a) providing a recombinant mussel byssus protein by the method of claim 11, and
b) (electro)spinning or molding said protein into threads by a suitable method.

## Patentansprüche

1. Hefezelle zur Herstellung eines rekombinanten, Collagen-ähnlichen Proteins, wobei die Hefezelle mit den folgenden Elementen transformiert worden ist:
a) einem ersten Expressionsvektor, welcher für das besagte rekombinante Collagen-ähnliche Protein kodiert; und
b) einem zweiten Expressionsvektor, der eine Nukleinsäure, die für Prolyl-4-hydroxylase (P4H) kodiert, umfasst,
wobei das rekombinante, Collagen-ähnliche Protein ein rekombinantes Muschelseide-Protein ist, welches ein Fragment oder mehrere Fragmente einer Muschel-Collagen-Domäne, flankiert von einer Muschel-Elastin-Domäne oder einer Muschel-Seidenfibroin-Domäne, umfasst oder welches aus einem Fragment oder mehreren Fragmenten einer Muschel-Collagen-Domäne, flankiert von einer Muschel-Elastin-Domäne oder einer Muschel-Seidenfibroin-Domäne, besteht.

2. Hefezelle nach Anspruch 1, wobei besagte P4H-Sequenz mit einer Signalsequenz zum effizienten Transport besagter Sequenz ins ER besagter Hefezelle verbunden ist, wobei die Signalsequenz vorzugsweise Mating Faktor Alpha 1 (MFa) von *S. serevisiae* ist.

3. Hefezelle nach Ansprüchen 1 oder 2, wobei die Hefezelle eine *S. serevisiae-, Schizosaccharomyces pombe-, Pichia pastoris-, Candida albicans-* oder *Hansenula polymorpha*-Zelle ist.

4. Hefezelle nach einem oder mehreren der Ansprüche 1 bis 3, wobei der erste Expressionsvektor ferner ein regulatorisches Element oder mehrere regulatorische Elemente umfasst, wobei das regulatorische Element oder die regulatorischen Elemente vorzugsweise einen Promoter umfasst/umfassen, der aus konstitutiven oder induzierbaren Promotoren ausgewählt ist.

5. Hefezelle nach Anspruch 4, wobei der Promoter aus GPD, Gal4, CUP1, MET25, GAL1 oder GAL1-10 ausgewählt ist.

6. Hefezelle nach einem oder mehreren der vorhergehenden Ansprüche, wobei die Expressionsvektoren Plasmide sind.

7. Hefezelle nach einem oder mehreren der vorhergehenden Ansprüche, wobei die Fragmente von *Mytilus sp.,* vorzugsweise *M*. *edulis, M. galloprovincialis, M. californians* oder *Geukeria demissa,* ausgewählt sind, oder wobei das rekombinante Muschelseide-Protein eines oder mehrere der Fragmente preColP und/oder preColD oder Varianten davon umfasst oder aus einem oder mehreren der Fragmente ColP und/oder preColD oder Varianten davon besteht.

8. Hefezelle nach einem oder mehreren der vorhergehenden Ansprüche, wobei das rekombinante Protein die Aminosäuresequenz SEQ ID NO: 3 und/oder SEQ ID NO: 4, oder Varianten davon umfasst oder daraus besteht.

9. Hefezelle nach einem oder mehreren der vorhergehenden Ansprüche, wobei in dem rekombinanten Protein die Signalsequenz der entsprechenden Aminosäuresequenz durch eine Hefe-spezifische Signalsequenz, vorzugsweise durch Mating Faktor Alpha 1 (MFa) von *S. serevisiae,* ersetzt wurde oder wobei die P4H eine humane P4H oder eine Muschel-P4H ist.

10. Kit of parts oder Co-Expressionssystem zur Verwendung bei der Herstellung eines rekombinanten Muschelseide-Proteins, welches die folgenden Komponenten umfasst:
a) den ersten Expressionsvektor, wie er in einem oder in mehreren der Ansprüche 1 bis 9 definiert ist, und
b) den zweiten Expressionsvektor, wie er in einem oder in mehreren der Ansprüche 1 bis 9 definiert ist.

11. Verfahren zur Herstellung eines rekombinanten Muschelseide-Proteins, umfassend die Schritte:
a) Zur-Verfügung-Stellen einer Hefezelle;
b) Transformieren besagter Hefezelle mit einem ersten und einem zweiten Expressionsvektor, wie er in einem oder in mehreren der Ansprüche 1 bis 9 definiert ist oder mit dem Co-Expressionssystem nach Anspruch 10;
c) Exprimieren des rekombinanten Muschelseide-Proteins in besagter Hefezelle unter geeigneten Bedingungen; und
d) Gewinnen des besagten rekombinanten Proteins.

12. Verfahren zur Herstellung von Fäden von einem rekombinanten Muschelseide-Proteins, umfassend die folgenden Schritte:
a) Zur-Verfügung-Stellen eines rekombinanten Muschelseide-Proteins, welches nach dem Verfahren nach Anspruch 11 hergestellt wurde, und
b) (Elektro)spinnen oder Formen besagten Proteins in Fäden durch ein geeignetes Verfahren.

## Revendications

1. Cellule de levure pour la production d'une protéine recombinante semblable au collagène, laquelle cellule de levure a été transformée avec les éléments suivants:
a) un premier vecteur d'expression qui code pour ladite protéine recombinante semblable au collagène; et
b) un deuxième vecteur d'expression comprenant un acide nucléique codant pour la propyl-4-hydroxylase (P4H),
tandis que la protéine recombinante semblable au collagène est une protéine recombinante du byssus de moule comprenant ou consistant en un ou plusieurs fragments d'un domaine de collagène de moule encadré par un domaine d'élastine de moule ou un domaine de fibroïne de soie de moule.

2. Cellule de levure selon la revendication 1, dans laquelle ladite séquence P4H est liée à une séquence signal pour le transport efficace de ladite séquence jusqu'au RE de ladite cellule de levure, tandis que la séquence signal est de préférence le facteur d'accouplement alpha 1 (MFa) de *S. cerevisiae.*

3. Cellule de levure selon les revendications 1 ou 2, dans laquelle la cellule de levure est une cellule de *S. cerevisiae, Schizosaccharomyces pombe*, *Pichia pastoris*, *Candida albicans,* ou *Hansenula polymorpha.*

4. Cellule de levure selon l'une ou plusieurs des revendications 1-3, dans laquelle le premier vecteur d'expression comprend en outre un ou plusieurs éléments régulateurs, tandis que les éléments régulateurs contiennent de préférence un promoteur choisi parmi les promoteurs constitutifs ou inductibles.

5. Cellule de levure selon la revendication 4, dans laquelle le promoteur est choisi parmi GPD, GAL4, CUP1, MET25, GAL1 ou GAL1-10.

6. Cellule de levure selon l'une ou plusieurs des revendications précédentes, dans laquelle les vecteurs d'expression sont des plasmides.

7. Cellule de levure selon l'une ou plusieurs des revendications précédentes, dans laquelle les fragments sont dérivés de *Mytilus sp.,* de préférence *M. edulis*, *M. galloprovincialis*, *M. californians*, ou *Geukeria demissa,* ou dans laquelle la protéine recombinante du byssus de moule comprend ou consiste en un ou plusieurs des fragments préColP et/ou préColD ou leurs variantes.

8. Cellule de levure selon l'une ou plusieurs des revendications précédentes, dans laquelle la protéine recombinante comprend ou consiste en la séquence d'acides aminés selon SEQ ID NO: 3 et/ou 4, ou leurs variantes.

9. Cellule de levure selon l'une ou plusieurs des revendications précédentes, dans laquelle dans la protéine recombinante la séquence signal de la séquence d'acides aminés correspondante est remplacée par une séquence signal spécifique pour la levure, de préférence par accouplement du facteur alpha 1 (MFa) de *S. cerevisiae*, ou dans laquelle le P4H est le P4H humain ou de moule.

10. Kit de parties ou système de co-expression pour utilisation dans la production d'une protéine recombinante du byssus de moule comprenant les constituants suivants:
a) le premier vecteur d'expression tel que défini dans l'une ou plusieurs des revendications 1-9; et
b) le deuxième vecteur d'expression tel que défini dans l'une ou plusieurs des revendications 1-9.

11. Procédé pour la production d'une protéine recombinante du byssus de moule, comprenant les étapes de:
a) fourniture d'une cellule de levure;
b) transformation de ladite cellule de levure avec un premier et un deuxième vecteur d'expression tels que définis dans l'une ou plusieurs des revendications 1-9 ou avec le système de co-expression selon la revendication 10;
c) expression de la protéine recombinante du byssus de moule à partir de ladite cellule de levure dans des conditions appropriées; et
d) récupération de ladite protéine recombinante.

12. Procédé pour la production de fils à partir d'une protéine recombinante du byssus de moule, comprenant les étapes suivantes:
a) fourniture d'une protéine recombinante du byssus de moule par le procédé selon la revendication 11, et
b) (électro)filage ou moulage de ladite protéine en des fils par un procédé approprié.


### SEQUENCE LISTING

<110> Technische Universitaet Muenchen
<120> Production of recombinant collagen like proteins
<130> P21539
<140>
   <141>
<150> EP 05025131.3
   <151> 2005-11-17
<160> 10
<170> PatentIn version 3.3
<210> 1
   <211> 2703
   <212> DNA
   <213> Mytilus edulis
<400> 1
   atggttcggt tctccctagc atcggtacta ttactggcag tcaccagcac agctttcgct      60
   ggaccagtta gtgattatgg tggtggtgga atcaaagtag taccctacca cggaggcgga     120
   ggtggaagcg gcggcggtgg cggtggaggc catggcggaa gcggtattgg tggtatcgga     180
   ggaggatcat cacatgcaca tgcccactct tcagcatctg cccatgtgca ccattttgga     240
   ccaggtggat cttcacatgc atcagctggt tcatcatccc atgcatccgc atcccataac     300
   ggtttaggag gtggcagtgc tcatgcacat agcagttcca gcgccaacgc tcattccggt     360
   ggattcggtg gattcggcgg tattggtggt attggcggta ttggcccagg aggaagtgtc     420
   ggaggcggta ttggcccagg aggaagtgtc ggaggcggca ttggcggtat tggcggtatt     480
   ggcggcggtg gtggaccagg cggtaatggc ggtatcggat tcggaccagg attcggagga     540
   ggattcggac caggttcatc tgctagtgga tccggaagtg gcagcgcatt cggtggtcca     600
   ggaggttcaa gcgcaagcgc aaacgcagct gcacgtgcaa atgcaaatgg tggtggagga     660
   ttcggtggac caggtacccc aggaaactca ggaccaccag gccaacccgg actaccagga     720
   gcaccaggcc aaccaggacg tccaggaagt accccaccag gtcgaccagg aaaccccgga     780
   ccaccaggtc aaccaggtaa cccaggacgt ccaggctctt caggaagacc aggaggatcc     840
   ggccaaccag gaggtccagg acgtccagga acccccggca aaccaggaaa ccgaggacaa     900
   ccaggacagc caggcggccc aggacaacca ggtcacccag gagcaggagg acaaccagga     960
   cgaaacggaa atccaggaaa ccccggtaaa ccaggaacac caggtcaccc aggaacagca    1020
   ggatcacgag gaatgccagg aaccccagga accccaggac aaccaggaat tccaggcacc    1080
   gtcggaggac gaggaccaag aggaccagct ggaatcatcg gattaattgg accaaaagga    1140
   aatccaggag agccaggaaa tccaggtgca ccaggaggcc caggatctac aggaccacaa    1200
   ggaccacaag gaccagccgg aggaccagga gcatcaggcg gaccaggaga caaaggcgca 1260
   ccaggtacac caggaggaac tggaccaaga ggaccaatcg gaccatcagg accatcagga 1320
   gcaccagggg accaaggacc acaaggaggt agaggaacac caggactcgc aggcaaacca 1380
   ggacctaaag gactacaagg atcaaatgga gaagttggac cccaaggacc atctggaccc 1440
   gcaggaccac aaggcccaca aggaaagaac ggtgtcaaag gagcagcagg agatcaagga 1500
   gctaggggac cagaaggaaa agccggacca gctggaccac aaggagaaac aggaccaaaa 1560
   ggaccaacag gagcacaagg accagccggt ccagccggac catcaggaga acaaggacca 1620
   ggaggggaaa gaggaggcca gggaccacaa ggagctgaag gaccaagtgg accagcagga 1680
   ccaagaggac cagcaggatc acaaggacca agtggtgaac gcggagaacc aggagcacca 1740
   ggtaaaaaag gaccaaatgg agaccgagga aaccaaggat caccaggagc accaggcaaa 1800
   aacggagcac gaggaaatag aggatcaaga ggaagcaacg gatcacccgg cagatcagga 1860
   tcaccaggaa gccgaggaaa accaggacca caaggaccac atggaccaag aggagcaaga 1920
   ggatcaccag gacaaaaagg accacgtgga gaccaaggag caccaggtgt tattcgtatt 1980
   gttatcgatg accagagaac aggaccagaa gttgcagaat tcccaggatt tggtggattc 2040
   ggaggagctt cagctaacgc agcaagttca gcaaatgcat ttgctggtgg acccggtggt 2100
   tccgctggag caggttcatc atcaggagct aacgcaaacg caggtggatt cccattcgga 2160
   ggaggaccat tcggaggagc aggaggtggt cccggagcag caggaggccc aggaggagca 2220
   ggaggcccag gaggagtagg aggaggagtt ggaggtggac caggaggagt aggaggtgga 2280
   gtaggaggtg gaccaggagg agtaggaggt ggaccaggag gagcaggacc aggaggagca 2340
   ggaggatttg gaccaggagg agcaggagga tttggtggat ttggaggagg atctagcgct 2400
   ggagcatcat catcaggatc agcatctgca tctaacggtg gaccattcgg agtactcaat 2460
   gtaggacccg gaggtagaat cggtggtgga agcgcatcag catctgcagc atctagagca 2520
   catgcacacg cttttggtgg tctcggaggg ggaagtgcct cagctggtag tcattcctca 2580
   tctagctcac actcatttgg cggacacgta ttccacagtg tgacccatca tggaggtcca 2640
   tcacatgttt caagcggagg tcacggaggt catggaggag gtccatacaa acctggatat 2700
   taa 2703
<210> 2
   <211> 2025
   <212> DNA
   <213> Mytilus edulis
<400> 2
   atggtctaca aactcctgac cgtgtgtctt gtagcatctc ttctagagat ttgcttagct 60
   gactataacg gcaacaaaca gtatggcggc agatacggca acagatacgg aaacggttta 120
   ggaggcggta atggtggtgc aggagccgta gcccatgccc atgcccatgc ccatgccagt 180
   gccggagcaa acggaagagc aagagcacat gcacgagcct tggcccatgc acatgccggt 240
   ggtggcgctg cacatggaca cccaggattc ccagttggtg gtagcgcaag cgcagccgca 300
   cgagcagcag cacgagcatc agcaggagga ttaggtggat tcggatcagc agcagccaat 360
   gcagcagcag cagcaagagc aggagcagga tttggtggat tcggtggatt aggaggattc 420
   ggaggactcg gaggagttgg cggtccaggt caaccaggac atgccggtaa acacggaacc 480
   gcaggagcag caggcaaagc aggacgtcca ggaccatgtg gagatagagg ggcaccagga 540
   gtaccaggca aacaaggacc agtaggagga caaggaccag caggaccacg aggaccacga 600
   ggagatgaag gaccagttgg accaaagggc gaaccaggag caagaggagc tgatggtaaa 660
   ccaggagaca aaggacctga tggagaaacc ggaccacaag gaccagctgg accaaaggga 720
   caagtaggag accaaggcaa accaggagca aagggagaaa ccggagatca aggagcacga 780
   ggtgaagcag gaaaggccgg cgaacaagga ccaggaggca tccaaggacc aaagggacca 840
   gtaggaggac aaggaccagc aggaccagcc ggaccactcg gaccacaagg accaatgggt 900
   gaacgaggac cacaaggacc aacaggatca gaaggaccag ttggagcacc aggaccaaag 960
   ggatcagtcg gagaccaagg agcacaagga gaccaaggag caactggcgc tgatggcaaa 1020
   aagggagaac caggagagag aggacaacaa ggagcagcag gaccagtcgg ccgaccagga 1080
   ccaagaggag atagaggagc aaagggaatt caaggaagcc gaggacgacc aggtggtatg 1140
   ggtagacgag gaaaccgtgg atcccaagga gcagtaggac cacgaggaga aactggccca 1200
   gacggtaacc aaggacaacg tggagaacaa ggagcaccag gagttatcac ccttgtcatt 1260
   gaagacctca gaacagccgg agtagaaagc cccgtagaaa cctttgacgc aggagcagga 1320
   accggtggac cagcaccagg agtaggagca gcagcaacag caggagcatt tgcaggagca 1380
   ggaccaggag gagctaatgc aggaggaaac gcagccgcag gagcaggacc aggagtagga 1440
   ccaggaggac tcggaggact aggaggactt ggtgcaggtg gactcggagg tggactcggc 1500
   ggtggactcg gaggattagg aggagcagga ggtttaggtg gtggactcgg aggattagga 1560
   ggaggtttag gtggtggact cggaggttta ggaggtggag caggaggagc aggaggcgca 1620
   ggagcaggag gaaacggtgg agcaggagca ggaggagcag gaggaaacgg tggaggatca 1680
   gccgcagcac gagcagcagc acaagcagca gcagcagcag gaggaaacgg tggagcagca 1740
   caagcagcag cacaagcagc agcatcagca gcagcaaatt caggacttgg agcaggagca 1800
   gcaagagcag cagcatcagc agccgctaga gcaaccgtag caggacatgg aagtggaacc 1860
   gccgcagcag cagccaacgc agccgcacaa gcacatgcag caacacgagg acaaggagga 1920
   tcacacgcac acgctgccgc cgcagctcac gcagccgcaa gtagcgtaat ccatggtggt 1980
   gactatcacg gaaacgatgc cggctatcac aaaccaggat attaa 2025
<210> 3
   <211> 900
   <212> PRT
   <213> Mytilus edulis
<400> 3
   Met Val Arg Phe Ser Leu Ala Ser Val Leu Leu Leu Ala Val Thr Ser
   1               5                   10                  15
   Thr Ala Phe Ala Gly Pro Val Ser Asp Tyr Gly Gly Gly Gly Ile Lys
               20                  25                  30
   Val Val Pro Tyr His Gly Gly Gly Gly Gly Ser Gly Gly Gly Gly Gly
           35                  40                  45
   Gly Gly His Gly Gly Ser Gly Ile Gly Gly Ile Gly Gly Gly Ser Ser
       50                  55                  60
   His Ala His Ala His Ser Ser Ala Ser Ala His Val His His Phe Gly
   65                  70                  75                  80
   Pro Gly Gly Ser Ser His Ala Ser Ala Gly Ser Ser Ser His Ala Ser
                   85                  90                  95
   Ala Ser His Asn Gly Leu Gly Gly Gly Ser Ala His Ala His Ser Ser
               100                 105                 110
   Ser Ser Ala Asn Ala His Ser Gly Gly Phe Gly Gly Phe Gly Gly Ile
           115                 120                 125
   Gly Gly Ile Gly Gly Ile Gly Pro Gly Gly Ser Val Gly Gly Gly Ile
       130                 135                 140
   Gly Pro Gly Gly Ser Val Gly Gly Gly Ile Gly Gly Ile Gly Gly Ile
   145                 150                 155                 160
   Gly Gly Gly Gly Gly Pro Gly Gly Asn Gly Gly Ile Gly Phe Gly Pro
                   165                 170                 175
   Gly Phe Gly Gly Gly Phe Gly Pro Gly Ser Ser Ala Ser Gly Ser Gly
               180                 185                 190
   Ser Gly Ser Ala Phe Gly Gly Pro Gly Gly Ser Ser Ala Ser Ala Asn
           195                 200                 205
   Ala Ala Ala Arg Ala Asn Ala Asn Gly Gly Gly Gly Phe Gly Gly Pro
   210 215 220
   Gly Thr Pro Gly Asn Ser Gly Pro Pro Gly Gln Pro Gly Leu Pro Gly
   225 230 235 240
   Ala Pro Gly Gln Pro Gly Arg Pro Gly Ser Thr Pro Pro Gly Arg Pro
   245 250 255
   Gly Asn Pro Gly Pro Pro Gly Gln Pro Gly Asn Pro Gly Arg Pro Gly
   260 265 270
   Ser Ser Gly Arg Pro Gly Gly Ser Gly Gln Pro Gly Gly Pro Gly Arg
   275 280 285
   Pro Gly Thr Pro Gly Lys Pro Gly Asn Arg Gly Gln Pro Gly Gln Pro
   290 295 300
   Gly Gly Pro Gly Gln Pro Gly His Pro Gly Ala Gly Gly Gln Pro Gly
   305 310 315 320
   Arg Asn Gly Asn Pro Gly Asn Pro Gly Lys Pro Gly Thr Pro Gly His
   325 330 335
   Pro Gly Thr Ala Gly Ser Arg Gly Met Pro Gly Thr Pro Gly Thr Pro
   340 345 350
   Gly Gln Pro Gly Ile Pro Gly Thr Val Gly Gly Arg Gly Pro Arg Gly
   355 360 365
   Pro Ala Gly Ile Ile Gly Leu Ile Gly Pro Lys Gly Asn Pro Gly Glu
   370 375 380
   Pro Gly Asn Pro Gly Ala Pro Gly Gly Pro Gly Ser Thr Gly Pro Gln
   385 390 395 400
   Gly Pro Gln Gly Pro Ala Gly Gly Pro Gly Ala Ser Gly Gly Pro Gly
   405 410 415
   Asp Lys Gly Ala Pro Gly Thr Pro Gly Gly Thr Gly Pro Arg Gly Pro
   420 425 430
   Ile Gly Pro Ser Gly Pro Ser Gly Ala Pro Gly Asp Gln Gly Pro Gln
   435 440 445
   Gly Gly Arg Gly Thr Pro Gly Leu Ala Gly Lys Pro Gly Pro Lys Gly
   450 455 460
   Leu Gln Gly Ser Asn Gly Glu Val Gly Pro Gln Gly Pro Ser Gly Pro
   465 470 475 480
   Ala Gly Pro Gln Gly Pro Gln Gly Lys Asn Gly Val Lys Gly Ala Ala
   485 490 495
   Gly Asp Gln Gly Ala Arg Gly Pro Glu Gly Lys Ala Gly Pro Ala Gly
   500 505 510
   Pro Gln Gly Glu Thr Gly Pro Lys Gly Pro Thr Gly Ala Gln Gly Pro
   515 520 525
   Ala Gly Pro Ala Gly Pro Ser Gly Glu Gln Gly Pro Gly Gly Glu Arg
   530 535 540
   Gly Gly Gln Gly Pro Gln Gly Ala Glu Gly Pro Ser Gly Pro Ala Gly
   545 550 555 560
   Pro Arg Gly Pro Ala Gly Ser Gln Gly Pro Ser Gly Glu Arg Gly Glu
   565 570 575
   Pro Gly Ala Pro Gly Lys Lys Gly Pro Asn Gly Asp Arg Gly Asn Gln
   580 585 590
   Gly Ser Pro Gly Ala Pro Gly Lys Asn Gly Ala Arg Gly Asn Arg Gly
   595 600 605
   Ser Arg Gly Ser Asn Gly Ser Pro Gly Arg Ser Gly Ser Pro Gly Ser
   610 615 620
   Arg Gly Lys Pro Gly Pro Gln Gly Pro His Gly Pro Arg Gly Ala Arg
   625 630 635 640
   Gly Ser Pro Gly Gln Lys Gly Pro Arg Gly Asp Gln Gly Ala Pro Gly
   645 650 655
   Val Ile Arg Ile Val Ile Asp Asp Gln Arg Thr Gly Pro Glu Val Ala
   660 665 670
   Glu Phe Pro Gly Phe Gly Gly Phe Gly Gly Ala Ser Ala Asn Ala Ala
   675 680 685
   Ser Ser Ala Asn Ala Phe Ala Gly Gly Pro Gly Gly Ser Ala Gly Ala
   690 695 700
   Gly Ser Ser Ser Gly Ala Asn Ala Asn Ala Gly Gly Phe Pro Phe Gly
   705 710 715 720
   Gly Gly Pro Phe Gly Gly Ala Gly Gly Gly Pro Gly Ala Ala Gly Gly
   725 730 735
   Pro Gly Gly Ala Gly Gly Pro Gly Gly Val Gly Gly Gly Val Gly Gly
   740 745 750
   Gly Pro Gly Gly Val Gly Gly Gly Val Gly Gly Gly Pro Gly Gly Val
   755 760 765
   Gly Gly Gly Pro Gly Gly Ala Gly Pro Gly Gly Ala Gly Gly Phe Gly
   770 775 780
   Pro Gly Gly Ala Gly Gly Phe Gly Gly Phe Gly Gly Gly Ser Ser Ala
   785 790 795 800
   Gly Ala Ser Ser Ser Gly Ser Ala Ser Ala Ser Asn Gly Gly Pro Phe
   805 810 815
   Gly Val Leu Asn Val Gly Pro Gly Gly Arg Ile Gly Gly Gly Ser Ala
   820 825 830
   Ser Ala Ser Ala Ala Ser Arg Ala His Ala His Ala Phe Gly Gly Leu
   835 840 845
   Gly Gly Gly Ser Ala Ser Ala Gly Ser His Ser Ser Ser Ser Ser His
   850 855 860
   Ser Phe Gly Gly His Val Phe His Ser Val Thr His His Gly Gly Pro
   865 870 875 880
   Ser His Val Ser Ser Gly Gly His Gly Gly His Gly Gly Gly Pro Tyr
   885 890 895
   Lys Pro Gly Tyr
   900
<210> 4
   <211> 674
   <212> PRT
   <213> Mytilus edulis
<400> 4
   Met Val Tyr Lys Leu Leu Thr Val Cys Leu Val Ala Ser Leu Leu Glu
   1               5                   10                  15
   Ile Cys Leu Ala Asp Tyr Asn Gly Asn Lys Gln Tyr Gly Gly Arg Tyr
               20                  25                  30
   Gly Asn Arg Tyr Gly Asn Gly Leu Gly Gly Gly Asn Gly Gly Ala Gly
   35 40 45
   Ala Val Ala His Ala His Ala His Ala His Ala Ser Ala Gly Ala Asn
   50 55 60
   Gly Arg Ala Arg Ala His Ala Arg Ala Leu Ala His Ala His Ala Gly
   65 70 75 80
   Gly Gly Ala Ala His Gly His Pro Gly Phe Pro Val Gly Gly Ser Ala
   85 90 95
   Ser Ala Ala Ala Arg Ala Ala Ala Arg Ala Ser Ala Gly Gly Leu Gly
   100 105 110
   Gly Phe Gly Ser Ala Ala Ala Asn Ala Ala Ala Ala Ala Arg Ala Gly
   115 120 125
   Ala Gly Phe Gly Gly Phe Gly Gly Leu Gly Gly Phe Gly Gly Leu Gly
   130 135 140
   Gly Val Gly Gly Pro Gly Gln Pro Gly His Ala Gly Lys His Gly Thr
   145 150 155 160
   Ala Gly Ala Ala Gly Lys Ala Gly Arg Pro Gly Pro Cys Gly Asp Arg
   165 170 175
   Gly Ala Pro Gly Val Pro Gly Lys Gln Gly Pro Val Gly Gly Gln Gly
   180 185 190
   Pro Ala Gly Pro Arg Gly Pro Arg Gly Asp Glu Gly Pro Val Gly Pro
   195 200 205
   Lys Gly Glu Pro Gly Ala Arg Gly Ala Asp Gly Lys Pro Gly Asp Lys
   210 215 220
   Gly Pro Asp Gly Glu Thr Gly Pro Gln Gly Pro Ala Gly Pro Lys Gly
   225 230 235 240
   Gln Val Gly Asp Gln Gly Lys Pro Gly Ala Lys Gly Glu Thr Gly Asp
   245 250 255
   Gln Gly Ala Arg Gly Glu Ala Gly Lys Ala Gly Glu Gln Gly Pro Gly
   260 265 270
   Gly Ile Gln Gly Pro Lys Gly Pro Val Gly Gly Gln Gly Pro Ala Gly
   275 280 285
   Pro Ala Gly Pro Leu Gly Pro Gln Gly Pro Met Gly Glu Arg Gly Pro
   290 295 300
   Gln Gly Pro Thr Gly Ser Glu Gly Pro Val Gly Ala Pro Gly Pro Lys
   305 310 315 320
   Gly Ser Val Gly Asp Gln Gly Ala Gln Gly Asp Gln Gly Ala Thr Gly
   325 330 335
   Ala Asp Gly Lys Lys Gly Glu Pro Gly Glu Arg Gly Gln Gln Gly Ala
   340 345 350
   Ala Gly Pro Val Gly Arg Pro Gly Pro Arg Gly Asp Arg Gly Ala Lys
   355 360 365
   Gly Ile Gln Gly Ser Arg Gly Arg Pro Gly Gly Met Gly Arg Arg Gly
   370 375 380
   Asn Arg Gly Ser Gln Gly Ala Val Gly Pro Arg Gly Glu Thr Gly Pro
   385 390 395 400
   Asp Gly Asn Gln Gly Gln Arg Gly Glu Gln Gly Ala Pro Gly Val Ile
   405 410 415
   Thr Leu Val Ile Glu Asp Leu Arg Thr Ala Gly Val Glu Ser Pro Val
   420 425 430
   Glu Thr Phe Asp Ala Gly Ala Gly Thr Gly Gly Pro Ala Pro Gly Val
   435 440 445
   Gly Ala Ala Ala Thr Ala Gly Ala Phe Ala Gly Ala Gly Pro Gly Gly
   450 455 460
   Ala Asn Ala Gly Gly Asn Ala Ala Ala Gly Ala Gly Pro Gly Val Gly
   465 470 475 480
   Pro Gly Gly Leu Gly Gly Leu Gly Gly Leu Gly Ala Gly Gly Leu Gly
   485 490 495
   Gly Gly Leu Gly Gly Gly Leu Gly Gly Leu Gly Gly Ala Gly Gly Leu
   500 505 510
   Gly Gly Gly Leu Gly Gly Leu Gly Gly Gly Leu Gly Gly Gly Leu Gly
   515 520 525
   Gly Leu Gly Gly Gly Ala Gly Gly Ala Gly Gly Ala Gly Ala Gly Gly
   530 535 540
   Asn Gly Gly Ala Gly Ala Gly Gly Ala Gly Gly Asn Gly Gly Gly Ser
   545 550 555 560
   Ala Ala Ala Arg Ala Ala Ala Gln Ala Ala Ala Ala Ala Gly Gly Asn
   565 570 575
   Gly Gly Ala Ala Gln Ala Ala Ala Gln Ala Ala Ala Ser Ala Ala Ala
   580 585 590
   Asn Ser Gly Leu Gly Ala Gly Ala Ala Arg Ala Ala Ala Ser Ala Ala
   595 600 605
   Ala Arg Ala Thr Val Ala Gly His Gly Ser Gly Thr Ala Ala Ala Ala
   610 615 620
   Ala Asn Ala Ala Ala Gln Ala His Ala Ala Thr Arg Gly Gln Gly Gly
   625 630 635 640
   Ser His Ala His Ala Ala Ala Ala Ala His Ala Ala Ala Ser Ser Val
   645 650 655
   Ile His Gly Gly Asp Tyr His Gly Asn Asp Ala Gly Tyr His Lys Pro
   660 665 670
   Gly Tyr
<210> 5
   <211> 536
   <212> PRT
   <213> Saccharomyces cerevisiae
<400> 5
   Met Arg Phe Pro Ser Ile Phe Thr Ala Val Leu Phe Ala Ala Ser Ser
   1 5 10 15
   Ala Leu Ala His Pro Gly Phe Phe Thr Ser Ile Gly Gln Met Thr Asp
   20 25 30
   Leu Ile His Thr Glu Lys Asp Leu Val Thr Ser Leu Lys Asp Tyr Ile
   35 40 45
   Lys Ala Glu Glu Asp Lys Leu Glu Gln Ile Lys Lys Trp Ala Glu Lys
   50 55 60
   Leu Asp Arg Leu Thr Ser Thr Ala Thr Lys Asp Pro Glu Gly Phe Val
   65 70 75 80
   Gly His Pro Val Asn Ala Phe Lys Leu Met Lys Arg Leu Asn Thr Glu
   85 90 95
   Trp Ser Glu Leu Glu Asn Leu Val Leu Lys Asp Met Ser Asp Gly Phe
   100 105 110
   Ile Ser Asn Leu Thr Ile Gln Arg Pro Val Leu Ser Asn Asp Glu Asp
   115 120 125
   Gln Val Gly Ala Ala Lys Ala Leu Leu Arg Leu Gln Asp Thr Tyr Asn
   130 135 140
   Leu Asp Thr Asp Thr Ile Ser Lys Gly Asn Leu Pro Gly Val Lys His
   145 150 155 160
   Lys Ser Phe Leu Thr Ala Glu Asp Cys Phe Glu Leu Gly Lys Val Ala
   165 170 175
   Tyr Thr Glu Ala Asp Tyr Tyr His Thr Glu Leu Trp Met Glu Gln Ala
   180 185 190
   Leu Arg Gln Leu Asp Glu Gly Glu Ile Ser Thr Ile Asp Lys Val Ser
   195 200 205
   Val Leu Asp Tyr Leu Ser Tyr Ala Val Tyr Gln Gln Gly Asp Leu Asp
   210 215 220
   Lys Ala Leu Leu Leu Thr Lys Lys Leu Leu Glu Leu Asp Pro Glu His
   225 230 235 240
   Gln Arg Ala Asn Gly Asn Leu Lys Tyr Phe Glu Tyr Ile Met Ala Lys
   245 250 255
   Glu Lys Asp Val Asn Lys Ser Ala Ser Asp Asp Gln Ser Asp Gln Lys
   260 265 270
   Thr Thr Pro Lys Lys Lys Gly Val Ala Val Asp Tyr Leu Pro Glu Arg
   275 280 285
   Gln Lys Tyr Glu Met Leu Cys Arg Gly Glu Gly Ile Lys Met Thr Pro
   290 295 300
   Arg Arg Gln Lys Lys Leu Phe Cys Arg Tyr His Asp Gly Asn Arg Asn
   305 310 315 320
   Pro Lys Phe Ile Leu Ala Pro Ala Lys Gln Glu Asp Glu Trp Asp Lys
                   325                 330                 335
   Pro Arg Ile Ile Arg Phe His Asp Ile Ile Ser Asp Ala Glu Ile Glu
               340                 345                 350
   Ile Val Lys Asp Leu Ala Lys Pro Arg Leu Ser Arg Ala Thr Val His
           355                 360                 365
   Asp Pro Glu Thr Gly Lys Leu Thr Thr Ala Gln Tyr Arg Val Ser Lys
       370                 375                 380
   Ser Ala Trp Leu Ser Gly Tyr Glu Asn Pro Val Val Ser Arg Ile Asn
   385                 390                 395                 400
   Met Arg Ile Gln Asp Leu Thr Gly Leu Asp Val Ser Thr Ala Glu Glu
                   405                 410                 415
   Leu Gln Val Ala Asn Tyr Gly Val Gly Gly Gln Tyr Glu Pro His Phe
               420                 425                 430
   Asp Phe Ala Arg Lys Asp Glu Pro Asp Ala Phe Lys Glu Leu Gly Thr
           435                 440                 445
   Gly Asn Arg Ile Ala Thr Trp Leu Phe Tyr Met Ser Asp Val Ser Ala
       450                 455                 460
   Gly Gly Ala Thr Val Phe Pro Glu Val Gly Ala Ser Val Trp Pro Lys
   465                 470                 475                 480
   Lys Gly Thr Ala Val Phe Trp Tyr Asn Leu Phe Ala Ser Gly Glu Gly
                   485                 490                 495
   Asp Tyr Ser Thr Arg His Ala Ala Cys Pro Val Leu Val Gly Asn Lys
               500                 505                 510
   Trp Val Ser Asn Lys Trp Leu His Glu Arg Gly Gln Glu Phe Arg Arg
           515                 520                 525
   Pro Cys Thr Leu Ser Glu Leu Glu
       530                 535
<210> 6
   <211> 510
   <212> PRT
   <213> Saccharomyces cerevisiae
<400> 6
   Met Arg Phe Pro Ser Ile Phe Thr Ala Val Leu Phe Ala Ala Ser Ser
   1 5 10 15
   Ala Leu Ala Asp Ala Pro Glu Glu Glu Asp His Val Leu Val Leu Arg
   20 25 30
   Lys Ser Asn Phe Ala Glu Ala Leu Ala Ala His Lys Tyr Leu Leu Val
   35 40 45
   Glu Phe Tyr Ala Pro Trp Cys Gly His Cys Lys Ala Leu Ala Pro Glu
   50 55 60
   Tyr Ala Lys Ala Ala Gly Lys Leu Lys Ala Glu Gly Ser Glu Ile Arg
   65 70 75 80
   Leu Ala Lys Val Asp Ala Thr Glu Glu Ser Asp Leu Ala Gln Gln Tyr
   85 90 95
   Gly Val Arg Gly Tyr Pro Thr Ile Lys Phe Phe Arg Asn Gly Asp Thr
   100 105 110
   Ala Ser Pro Lys Glu Tyr Thr Ala Gly Arg Glu Ala Asp Asp Ile Val
   115 120 125
   Asn Trp Leu Lys Lys Arg Thr Gly Pro Ala Ala Thr Thr Leu Pro Asp
   130 135 140
   Gly Ala Ala Ala Glu Ser Leu Val Glu Ser Ser Glu Val Ala Val Ile
   145 150 155 160
   Gly Phe Phe Lys Asp Val Glu Ser Asp Ser Ala Lys Gln Phe Leu Gln
   165 170 175
   Ala Ala Glu Ala Ile Asp Asp Ile Pro Phe Gly Ile Thr Ser Asn Ser
   180 185 190
   Asp Val Phe Ser Lys Tyr Gln Leu Asp Lys Asp Gly Val Val Leu Phe
   195 200 205
   Lys Lys Phe Asp Glu Gly Arg Asn Asn Phe Glu Gly Glu Val Thr Lys
   210 215 220
   Glu Asn Leu Leu Asp Phe Ile Lys His Asn Gln Leu Pro Leu Val Ile
   225 230 235 240
   Glu Phe Thr Glu Gln Thr Ala Pro Lys Ile Phe Gly Gly Glu Ile Lys
   245 250 255
   Thr His Ile Leu Leu Phe Leu Pro Lys Ser Val Ser Asp Tyr Asp Gly
           260                 265                 270
   Lys Leu Ser Asn Phe Lys Thr Ala Ala Glu Ser Phe Lys Gly Lys Ile
       275                 280                 285
   Leu Phe Ile Phe Ile Asp Ser Asp His Thr Asp Asn Gln Arg Ile Leu
   290                 295                 300
   Glu Phe Phe Gly Leu Lys Lys Glu Glu Cys Pro Ala Val Arg Leu Ile
   305                 310                 315                 320
   Thr Leu Glu Glu Glu Met Thr Lys Tyr Lys Pro Glu Ser Glu Glu Leu
                   325                 330                 335
   Thr Ala Glu Arg Ile Thr Glu Phe Cys His Arg Phe Leu Glu Gly Lys
               340                 345                 350
   Ile Lys Pro His Leu Met Ser Gln Glu Leu Pro Glu Asp Trp Asp Lys
           355                 360                 365
   Gln Pro Val Lys Val Leu Val Gly Lys Asn Phe Glu Asp Val Ala Phe
       370                 375                 380
   Asp Glu Lys Lys Asn Val Phe Val Glu Phe Tyr Ala Pro Trp Cys Gly
   385                 390                 395                 400
   His Cys Lys Gln Leu Ala Pro Ile Trp Asp Lys Leu Gly Glu Thr Tyr
                   405                 410                 415
   Lys Asp His Glu Asn Ile Val Ile Ala Lys Met Asp Ser Thr Ala Asn
               420                 425                 430
   Glu Val Glu Ala Val Lys Val His Ser Phe Pro Thr Leu Lys Phe Phe
           435                 440                 445
   Pro Ala Ser Ala Asp Arg Thr Val Ile Asp Tyr Asn Gly Glu Arg Thr
       450                 455                 460
   Leu Asp Gly Phe Lys Lys Phe Leu Glu Ser Gly Gly Gln Asp Gly Ala
   465                 470                 475                 480
   Gly Asp Asp Asp Asp Leu Glu Asp Leu Glu Glu Ala Glu Glu Pro Asp
                   485                 490                 495
   Met Glu Glu Asp Asp Asp Gln Lys Ala Val Lys Asp Glu Leu
               500                 505                 510
<210> 7
   <211> 3876
   <212> DNA
   <213> Mytilus edulis
<400> 7
   ccgcggtcat tacagttcat ctttcacagc tttctgatca tcgtcttcct ccatgtctgg 60
   ctcctctgct tcttccaggt cctcgagatc gtcatcatcc cctgccccat cctggccacc 120
   cgagaggtcc ttaaagaatt ttggtaggtc gcacgcaagg ggcaacatta gttactggca 180
   cctgtcggca ctggcaggaa agaacttgag tgtggggaag ctgtgcactt tgacggcctc 240
   cacctcgttg gcagtcgagt ccatcttggc gatgacgatg ttctcatggt ccttgtacgt 300
   ctctcccagt ttatcccaaa tgggagccaa ctgtttgcag tgaccacacc atggggcata 360
   gaactccaca aagacgtttt ttttctcatc aaaagccacg tcttcaaagt tcttcccaac 420
   aagcaccttg acaggctgct tgtcccagtc ctccggcagc tcctggctca tcaggtgggg 480
   cttgattttg ccctccagga agcggtggca gaactctgtg atcctctctg ccgtcagctc 540
   ctccgattcg ggcttgtact tggtcatctc ctcctccagg gtgatgaggc gcacggccgg 600
   gcactcttcc ttcttcaggc caaagaactc gaggatgcgc tggttgtcgg tgtggtcgct 660
   gtcgatgaag atgaacagga tcttgccctt gaagctctcg gctgctgttt tgaagttgct 720
   cagtttgccg tcatagtcag acacactctt gggcaagaac agcaggatgt gagtcttgat 780
   ttcacctcca aaaatcttcg gggctgtctg ctcggtgaac tcgatgacaa ggggcagctg 840
   gttgtgtttg ataaagtcca gcaggttctc cttggtgacc tccccttcaa agttgttccg 900
   gccttcatca aacttcttaa agaggacaac cccatctttg tcgagctggt atttggagaa 960
   cacgtcactg ttggaagtga tcccaaatgg tatgtcatcg atggcctctg ctgcctgcaa 1020
   aaactgcttg gcagagtccg actccacgtc cttgaagaag ccgatgacag ccacctcgct 1080
   ggactccacc aaggactctg cagctgcgcc gtcaggcagg gtggtggcag ccgggcccgt 1140
   gcgcttcttc agccagttca cgatgtcatc agcctctctg ccagctgtat attccttggg 1200
   ggaagccgtg tctccattcc tgaagaactt gatggtggga tagccgcgca cgccgtactg 1260
   ctgggccagg tcagactcct ccgtggcgtc caccttggcc aacctgatct cggaaccttc 1320
   tgccttcagc ttcccagcgg ctttggcata ctcaggggcc agagccttgc agtggccaca 1380
   ggttccccgt atcttgaggt ggtcgtccat gaacacccgg cggtcgcgga ggcgcttcaa 1440
   gcttttccgc agcaccagga cgtggtcctc ctcctccgga gcgtcagcta atgcggagga 1500
   tgctgcgaat aaaactgcag taaaaattga aggaaatctc atggatccgg ggttttttct 1560
   ccttgacgtt aaagtataga ggtatattaa caattttttg ttgatacttt tattacattt 1620
   gaataagaag taatacaaac cgaaaatgtt gaaagtatta gttaaagtgg ttatgcagtt 1680
   tttgcattta tatatctgtt aatagatcaa aaatcatcgc ttcgctgatt aattacccca   1740
   gaaataaggc taaaaaacta atcgcattat catcctatgg ttgttaattt gattcgttca   1800
   tttgaaggtt tgtggggcca ggttactgcc aatttttcct cttcataacc ataaaagcta   1860
   gtattgtaga atctttattg ttcggagcag tgcggcgcga ggcacatctg cgtttcagga   1920
   acgcgaccgg tgaagacgag gacgcacgga ggagagtctt ccttcggagg gctgtcaccc   1980
   gctcggcggc ttctaatccg tacttcaata tagcaatgag cagttaagcg tattactgaa   2040
   agttccaaag agaaggtttt tttaggctaa gataatgggg ctctttacat ttccacaaca   2100
   tataagtaag attagatatg gatatgtata tggatatgta tatggtggta atgccatgta   2160
   atatgattat taaacttctt tgcgtccatc caaaaaaaaa gtaagaattt ttgaaaattc   2220
   aaggaattcg atatcaagct tatcgatacc gtcgacatga gatttccttc aatttttact   2280
   gcagttttat tcgcagcatc ctccgcgcta gctcatccag gcttttttac ttcaattggt   2340
   cagatgactg atttgatcca tactgagaaa gatctggtga cttctctgaa agattatatt   2400
   aaggcagaag aggacaagtt agaacaaata aaaaaatggg cagagaagtt agatcggcta   2460
   actagtacag cgacaaaaga tccagaagga tttgttgggc atccagtaaa tgcattcaaa   2520
   ttaatgaaac gtctgaatac tgagtggagt gagttggaga atctggtcct taaggatatg   2580
   tcagatggct ttatctctaa cctaaccatt cagagaccag tactttctaa tgatgaagat   2640
   caggttgggg cagccaaagc tctgttacgt ctccaggata cctacaattt ggatacagat   2700
   accatctcaa agggtaatct tccaggagtg aaacacaaat cttttctaac ggctgaggac   2760
   tgctttgagt tgggcaaagt ggcctataca gaagcagatt attaccatac ggaactgtgg   2820
   atggaacaag ccctaaggca actggatgaa ggcgagattt ctaccataga taaagtctct   2880
   gttctagatt atttgagcta tgcggtatat cagcagggag acctggataa ggcacttttg   2940
   ctcacaaaga agcttcttga actagatcct gaacatcaga gagctaatgg taacttaaaa   3000
   tattttgagt atataatggc taaagaaaaa gatgtcaata agtctgcttc agatgaccaa   3060
   tctgatcaga aaactacacc aaagaaaaaa ggggttgctg tggattacct gccagagaga   3120
   cagaagtacg aaatgctgtg ccgtggggag ggtatcaaaa tgacccctcg gagacagaaa   3180
   aaactctttt gccgctacca tgatggaaac cgtaatccta aatttattct ggctccagct   3240
   aaacaggagg atgaatggga caagcctcgt attattcgct tccatgatat tatttctgat   3300
   gcagaaattg aaatcgtcaa agacctagca aaaccaaggc tgagccgagc tacagtacat   3360
   gaccctgaga ctggaaaatt gaccacagca cagtacagag tatctaagag tgcctggctc   3420
   tctggctatg aaaatcctgt ggtgtctcga attaatatga gaatacaaga tctaacagga   3480
   ctagatgttt ccacagcaga ggaattacag gtagcaaatt atggagttgg aggacagtat   3540
   gaaccccatt ttgactttgc acggaaagat gagccagatg ctttcaaaga gctggggaca 3600
   ggaaatagaa ttgctacatg gctgttttat atgagtgatg tgtctgcagg aggagccact 3660
   gtttttcctg aagttggagc tagtgtttgg cccaaaaaag gaactgctgt tttctggtat 3720
   aatctgtttg ccagtggaga aggagattat agtacacggc atgcagcctg tccagtgcta 3780
   gttggcaaca aatgggtatc caataaatgg ctccatgaac gtggacaaga atttcgaaga 3840
   ccttgtacgt tgtcagaatt ggaatgatga gggccc 3876
<210> 8
   <211> 922
   <212> PRT
   <213> Mytilus edulis
<220>
   <221> misc_feature
   <222> (307)..(307)
   <223> Xaa can be any naturally occurring amino acid
<400> 8
   Met Val Tyr Lys Leu Leu Thr Val Cys Leu Val Ala Ser Leu Leu Glu
   1               5                   10                  15
   Ile Cys Leu Ala Asp Tyr Asn Gly Asn Lys Gln Tyr Gly Gly Arg Tyr
               20                  25                  30
   Gly Asn Arg Tyr Gly Asn Gly Leu Gly Gly Gly Asn Gly Gly Ala Gly
           35                  40                  45
   Ala Val Ala His Ala His Ala His Ala His Ala Ser Ala Gly Ala Asn
       50                  55                  60
   Gly Arg Ala Arg Ala His Ala Arg Ala Leu Ala His Ala His Ala Gly
   65                  70                  75                  80
   Gly Gly Ala Ala His Gly His Pro Gly Phe Pro Val Gly Gly Ser Ala
                   85                  90                  95
   Ser Ala Ala Ala Arg Ala Ala Ala Arg Ala Ser Ala Gly Gly Leu Gly
               100                 105                 110
   Gly Phe Gly Ser Ala Ala Ala Asn Ala Ala Ala Ala Ala Arg Ala Gly
           115                 120                 125
   Ala Gly Phe Gly Gly Phe Gly Gly Leu Gly Gly Phe Gly Gly Leu Gly
       130                 135                 140
   Gly Val Gly Gly Pro Gly Gln Pro Gly Gly Pro Gly Gly Pro Gly Gly
   145 150 155 160
   Pro Gly Gly Pro Gly Gly Pro Gly Met Pro Gly Gly Pro Gly Gly Pro
   165 170 175
   Ser Gly Pro Gly Thr Gly Gly Pro Gly Gln Pro Gly Gly Pro Gly Gly
   180 185 190
   Pro Gly Gly Pro Gly Gly Pro Gly Gly Pro Ser Met Pro Gly Gly Pro
   195 200 205
   Gly Gly Pro Gly Gly Pro Gly Met Pro Gly Gly Pro Gly Gly Pro Gly
   210 215 220
   Gly Pro Gly Gly Ala Gly Gly Ile Pro Gly Met Thr Gly Pro Ala Gly
   225 230 235 240
   Pro Pro Gly Pro Ala Gly Pro Gln Gly Pro Glu Gly Glu Gln Gly Pro
   245 250 255
   Arg Gly Arg Thr Pro Ala Gly Thr Pro Gly Pro Pro Gly Asn Pro Gly
   260 265 270
   Glu Pro Gly Gln Gly Gly Ala Pro Gly Ala Pro Gly Ala Pro Gly His
   275 280 285
   Ala Gly Lys His Gly Thr Ala Gly Ala Ala Gly Lys Ala Gly Arg Pro
   290 295 300
   Gly Pro Xaa Gly Gln Ala Gly Ala Ser Gly Ser Ser Gly Gln His Gly
   305 310 315 320
   Ala Ser Gly Ala Pro Gly Arg Pro Gly Asn Pro Gly Ser Thr Gly Arg
   325 330 335
   Pro Gly Ala Thr Gly Asp Pro Gly Arg Pro Gly Ala Thr Gly Thr Thr
   340 345 350
   Gly Arg Pro Gly Pro Ser Gly Ala Pro Gly Asn Pro Gly Ala Pro Gly
   355 360 365
   Ala Leu Gly Ala Pro Gly Pro Arg Gly Ser Pro Gly Phe Val Gly Leu
   370 375 380
   Pro Gly Pro Arg Gly Ser Pro Gly Glu Pro Gly Asn Gln Gly Pro Ile
   385 390 395 400
   Gly Gly Pro Gly Tyr Pro Gly Pro Arg Gly Pro Gln Gly Pro Asp Gly
   405 410 415
   Ala Met Gly Pro Gln Gly Pro Cys Gly Asp Arg Gly Ala Pro Gly Val
   420 425 430
   Pro Gly Lys Gln Gly Pro Val Gly Gly Gln Gly Pro Ala Gly Pro Arg
   435 440 445
   Gly Pro Arg Gly Asp Glu Gly Pro Val Gly Pro Lys Gly Glu Pro Gly
   450 455 460
   Ala Arg Gly Ala Asp Gly Lys Pro Gly Asp Lys Gly Pro Asp Gly Glu
   465 470 475 480
   Thr Gly Pro Gln Gly Pro Ala Gly Pro Lys Gly Gln Val Gly Asp Gln
   485 490 495
   Gly Lys Pro Gly Ala Lys Gly Glu Thr Gly Asp Gln Gly Ala Arg Gly
   500 505 510
   Glu Ala Gly Lys Ala Gly Glu Gln Gly Pro Gly Gly Ile Gln Gly Pro
   515 520 525
   Lys Gly Pro Val Gly Gly Gln Gly Pro Ala Gly Pro Ala Gly Pro Leu
   530 535 540
   Gly Pro Gln Gly Pro Met Gly Glu Arg Gly Pro Gln Gly Pro Thr Gly
   545 550 555 560
   Ser Glu Gly Pro Val Gly Ala Pro Gly Pro Lys Gly Ser Val Gly Asp
   565 570 575
   Gln Gly Ala Gln Gly Asp Gln Gly Ala Thr Gly Ala Asp Gly Lys Lys
   580 585 590
   Gly Glu Pro Gly Glu Arg Gly Gln Gln Gly Ala Ala Gly Pro Val Gly
   595 600 605
   Arg Pro Gly Pro Arg Gly Asp Arg Gly Ala Lys Gly Ile Gln Gly Ser
   610 615 620
   Arg Gly Arg Pro Gly Gly Met Gly Arg Arg Gly Asn Arg Gly Ser Gln
   625 630 635 640
   Gly Ala Val Gly Pro Arg Gly Glu Thr Gly Pro Asp Gly Asn Gln Gly
   645 650 655
   Gln Arg Gly Glu Gln Gly Ala Pro Gly Val Ile Thr Leu Val Ile Glu
   660 665 670
   Asp Leu Arg Thr Ala Gly Val Glu Ser Pro Val Glu Thr Phe Asp Ala
   675 680 685
   Gly Ala Gly Thr Gly Gly Pro Ala Pro Gly Val Gly Ala Ala Ala Thr
   690 695 700
   Ala Gly Ala Phe Ala Gly Ala Gly Pro Gly Gly Ala Asn Ala Gly Gly
   705 710 715 720
   Asn Ala Ala Ala Gly Ala Gly Pro Gly Val Gly Pro Gly Gly Leu Gly
   725 730 735
   Gly Leu Gly Gly Leu Gly Ala Gly Gly Leu Gly Gly Gly Leu Gly Gly
   740 745 750
   Gly Leu Gly Gly Leu Gly Gly Ala Gly Gly Leu Gly Gly Gly Leu Gly
   755 760 765
   Gly Leu Gly Gly Gly Leu Gly Gly Gly Leu Gly Gly Leu Gly Gly Gly
   770 775 780
   Ala Gly Gly Ala Gly Ala Gly Gly Asn Gly Gly Ala Gly Ala Gly Gly
   785 790 795 800
   Ala Gly Gly Asn Gly Gly Gly Ser Ala Ala Ala Arg Ala Ala Ala Gln
   805 810 815
   Ala Ala Ala Ala Ala Gly Gly Asn Gly Gly Ala Ala Gln Ala Ala Ala
   820 825 830
   Gln Ala Ala Ala Ser Ala Ala Ala Asn Ser Gly Leu Gly Ala Gly Ala
   835 840 845
   Ala Arg Ala Ala Ala Ser Ala Ala Ala Arg Ala Thr Val Thr Gly His
   850 855 860
   Gly Ser Gly Thr Ala Ala Ala Ala Ala Asn Ala Ala Ala Gln Ala His
   865 870 875 880
   Ala Ala Thr Arg Gly Gln Gly Gly Ser His Ala His Ala Ala Ala Ala
   885 890 895
   Ala His Ala Ala Ala Ser Ser Val Ile His Gly Gly Asp Tyr His Gly
   900 905 910
   Asn Asp Ala Gly Tyr His Lys Pro Gly Tyr
   915 920
<210> 9
   <211> 19
   <212> PRT
   <213> Saccharomyces cerevisiae
<400> 9 Ala Leu Ala
<210> 10
   <211> 952
   <212> PRT
   <213> Mytilus edulis
<400> 10
   Met Val Arg Phe Ser Leu Ala Ser Val Leu Leu Leu Ala Val Thr Ser
   1 5 10 15
   Thr Ala Phe Ala Gly Pro Val Ser Asp Tyr Gly Gly Gly Gly Ile Lys
   20 25 30
   Val Val Pro Tyr His Gly Gly Gly Gly Gly Gly Gly Gly Gly Gly Gly
   35 40 45
   Gly Gly His Gly Gly Ser Phe Arg Asn Gly Arg His Gly Gly Ile Gly
   50 55 60
   Gly Ile Gly Gly Gly Ser Ser His Ala His Ala His Ser Ser Ala Ser
   65 70 75 80
   Ala His Val His His Phe Gly Pro Gly Gly Ser Ser His Ala Ser Ala
   85 90 95
   Gly Ser Ser Ser His Ala Ser Ala Ser His Asn Gly Leu Gly Gly Gly
   100 105 110
   Ser Ala His Ala His Ser Ser Ser Ser Ala Asn Ala His Ser Gly Gly
   115 120 125
   Phe Gly Gly Phe Gly Gly Ile Gly Gly Ile Gly Gly Ile Gly Pro Gly
   130 135 140
   Gly Ser Val Gly Gly Gly Ile Gly Pro Gly Gly Ser Val Gly Gly Gly
   145 150 155 160
   Ile Gly Gly Ile Gly Gly Ile Gly Gly Gly Gly Gly Pro Gly Gly Asn
   165 170 175
   Gly Gly Ile Gly Phe Gly Pro Gly Phe Gly Gly Gly Phe Gly Pro Gly
   180 185 190
   Ser Ser Ala Ser Gly Ser Gly Ser Gly Ser Ala Phe Gly Gly Pro Gly
   195 200 205
   Gly Ser Ser Ala Ser Ala Asn Ala Ala Ala Arg Ala Asn Ala Asn Gly
   210 215 220
   Gly Gly Gly Phe Gly Gly Pro Gly Thr Pro Gly Asn Ser Gly Pro Pro
   225 230 235 240
   Gly Gln Pro Gly Leu Pro Gly Ala Pro Gly Gln Pro Gly Arg Pro Gly
   245 250 255
   Ser Thr Pro Pro Gly Arg Leu Gly Asn Pro Gly Pro Pro Gly Gln Pro
   260 265 270
   Gly Asn Pro Gly Arg Pro Gly Ser Ser Gly Arg Pro Gly Gly Ser Gly
   275 280 285
   Gln Pro Gly Gly Pro Gly Arg Pro Gly Thr Pro Gly Lys Pro Gly Asn
   290 295 300
   Arg Gly Gln Pro Gly Gln Pro Gly Gly Pro Gly Gln Pro Gly His Pro
   305 310 315 320
   Gly Ala Gly Gly Gln Pro Gly Arg Asn Gly Asn Pro Gly Asn Pro Gly
   325 330 335
   Lys Pro Gly Thr Pro Gly His Pro Gly Thr Ala Gly Ser Arg Gly Met
   340 345 350
   Pro Gly Thr Pro Gly Thr Pro Gly Gln Pro Gly Ile Pro Gly Thr Val
   355 360 365
   Gly Gly Arg Gly Pro Arg Gly Pro Ala Gly Ile Ile Gly Leu Ile Gly
   370 375 380
   Pro Lys Gly Asn Pro Gly Glu Pro Gly Asn Pro Gly Ala Pro Gly Gly
   385 390 395 400
   Pro Gly Ser Thr Gly Pro Gln Gly Pro Gln Gly Pro Ala Gly Gly Pro
   405 410 415
   Gly Ala Ser Gly Gly Pro Gly Asp Lys Gly Ala Pro Gly Thr Pro Gly
   420 425 430
   Gly Thr Gly Pro Arg Gly Pro Ile Gly Pro Ser Gly Pro Ser Gly Ala
   435 440 445
   Pro Gly Asp Gln Gly Pro Gln Gly Gly Arg Gly Thr Pro Gly Leu Ala
   450 455 460
   Gly Lys Pro Gly Pro Lys Gly Leu Gln Gly Ser Asn Gly Glu Val Gly
   465 470 475 480
   Pro Gln Gly Pro Ser Gly Pro Ala Gly Pro Gln Gly Pro Gln Gly Lys
   485 490 495
   Asn Gly Val Lys Gly Ala Ala Gly Asp Gln Gly Ala Arg Gly Pro Glu
   500 505 510
   Gly Lys Ala Gly Pro Ala Gly Pro Gln Gly Glu Thr Gly Pro Lys Gly
   515 520 525
   Pro Thr Gly Ala Gln Gly Pro Ala Gly Pro Ala Gly Pro Ser Gly Glu
   530 535 540
   Gln Gly Pro Gly Gly Glu Arg Gly Gly Gln Gly Pro Gln Gly Ala Glu
   545 550 555 560
   Gly Pro Ser Gly Pro Ala Gly Pro Arg Gly Pro Ala Gly Ser Gln Gly
   565 570 575
   Pro Ser Gly Glu Arg Gly Glu Pro Gly Ala Pro Gly Lys Lys Gly Pro
   580 585 590
   Asn Gly Asp Arg Gly Asn Gln Gly Ser Pro Gly Ala Pro Gly Lys Asn
   595 600 605
   Gly Ala Arg Gly Asn Arg Gly Ser Arg Gly Ser Asn Gly Ser Pro Gly
   610 615 620
   Arg Ser Gly Ser Pro Gly Ser Arg Gly Lys Pro Gly Pro Gln Gly Pro
   625 630 635 640
   His Gly Pro Arg Gly Leu Arg Gly Ser Pro Gly Gln Lys Gly Pro Arg
   645 650 655
   Gly Asp Gln Gly Ala Pro Gly Val Ile Arg Ile Val Ile Asp Asp Gln
   660 665 670
   Arg Thr Gly Pro Glu Val Ala Glu Phe Pro Gly Phe Gly Gly Phe Gly
   675 680 685
   Gly Ala Ser Ala Asn Ala Ala Ser Ser Ala Asn Ala Phe Ala Gly Gly
   690 695 700
   Pro Gly Gly Ser Ala Gly Ala Gly Ser Ser Ser Gly Ala Asn Ala Asn
   705 710 715 720
   Ala Gly Gly Phe Pro Phe Gly Gly Ala Gly Gly Gly Pro Gly Ala Ala
   725 730 735
   Gly Gly Pro Gly Gly Ala Gly Gly Pro Gly Gly Val Gly Gly Gly Val
   740 745 750
   Gly Gly Gly Pro Gly Gly Val Gly Gly Gly Val Gly Gly Gly Pro Gly
   755 760 765
   Gly Val Gly Gly Gly Pro Gly Gly Ala Gly Pro Gly Gly Ala Gly Gly
   770 775 780
   Phe Gly Pro Gly Gly Ala Gly Gly Phe Gly Gly Phe Gly Gly Gly Ser
   785 790 795 800
   Ser Ala Gly Ala Ser Ser Ser Gly Ser Ala Ser Ala Ser Asn Gly Gly
   805 810 815
   Pro Phe Gly Val Leu Asn Val Gly Pro Gly Gly Arg Ile Gly Gly Gly
   820 825 830
   Ser Ala Ser Ala Ser Ala Ala Ser Arg Ala His Ala His Phe Gly Gly
   835 840 845
   Gly Ser Ser Ala Gly Ala Ser Ser Ser Gly Ser Ala Ser Ala Ser Asn
   850 855 860
   Gly Gly Pro Phe Gly Val Leu Asn Val Gly Pro Gly Gly Arg Ile Gly
   865 870 875 880
   Gly Gly Ser Ala Ser Ala Ser Ala Ala Ser Arg Ala His Ala His Ala
   885 890 895
   Phe Gly Gly Leu Gly Gly Gly Ser Ala Ser Ala Gly Ser His Ser Ser
               900                 905                 910
   Ser Ser Ser His Ser Phe Gly Gly His Val Phe His Ser Val Thr His
           915                 920                 925
   His Gly Gly Pro Ser His Val Ser Ser Gly Gly His Gly Gly His Gly
       930                 935                 940
   Gly Gly Pro Tyr Lys Pro Gly Tyr
   945                 950